# EUROPEAN PATENT APPLICATION

(11) **EP 4 799 730 A2**
(43) Date of publication of application: **02.09.2026**
(21) Application number: 26175960.9
(22) Date of filing: 26.10.2021
(51) Int. Cl.: B01L 3/00

(54) **SINGLE MOLECULE DETECTION SYSTEM USING PHOTOBLEACHING INFORMATION**

(30) Priority: 27.10.2020 US 202063106325 P; 25.03.2021 US 202163165798 P; 06.10.2021 US 202163252906 P
(62) Document number(s) of the earlier application(s) in accordance with Art. 76 EPC: 21887371.9 / 4 221 894
(71) Applicant: Quantum-Si Incorporated, Branford, CT 06405 (US)
(72) Inventor: PRESTON, Kyle, Guilford, 06437 (US); REED, Brian, Madison, 06443 (US); SCHMID, Gerard, Guilford, 06437 (US); SHI, Xinghua, Madison, 06443 (US); HUANG, Haidong, Madison, 06443 (US)
(74) Representative: Carpmaels & Ransford LLP

(57) **Abstract**

Aspects of the present disclosure relate to techniques for calibrating a system comprising integrated device. According to some embodiments, there is provided a method for calibrating a system comprising an integrated device, the method comprising: exciting, with light from at least one excitation source, a reference dye molecule disposed in a chamber of the integrated device; obtaining a signal emitted by the reference dye molecule, the signal containing information representative of a bleaching time of the reference dye molecule; and adjusting one or more characteristics of the system based on the bleaching time of the reference dye molecule.

## Description

### CROSS-REFERENCE TO RELATED APPLICATIONS

This application claims the benefit under 35 U.S.C. § 119(e) of U.S. Provisional Patent Application Serial No. 63/106,325 titled "SINGLE-MOLECULE DETECTION SYSTEM USING PHOTOBLEACHING INFORMATION" filed on October 27, 2020 under Attorney Docket No. R0708.70114US00, U.S. Provisional Patent Application Serial No. 63/165,798 titled "SINGLE-MOLECULE DETECTION SYSTEM CALIBRATION USING PHOTOBLEACHING INFORMATION" filed on March 25, 2021 under Attorney Docket No. R0708.70114US01, and U.S. Provisional Application Serial No. 63/252,906 titled "SYSTEMS AND METHODS FOR DETERMINING QUANTITATIVE LOADING OF AN INTEGRATED DEVICE" filed on October 6, 2021 under Attorney Docket No. R0708.70114US02, each of which is hereby incorporated by reference in their entireties herein.

### FIELD OF THE DISCLOSURE

The present disclosure relates to integrated devices and related instruments that can perform massively-parallel analyses of samples by providing short optical pulses to tens of thousands of reaction chambers or more simultaneously and receiving fluorescent signals from the reaction chambers for sample analyses. The instruments may be useful for point-of-care genetic sequencing and for personalized medicine.

### BACKGROUND

Photodetectors are used to detect light in a variety of applications. Integrated photodetectors have been developed that produce an electrical signal indicative of the intensity of incident light. Integrated photodetectors for imaging applications include an array of pixels to detect the intensity of light received from across a scene. Examples of integrated photodetectors include charge coupled devices (CCDs) and Complementary Metal Oxide Semiconductor (CMOS) image sensors.

Instruments that are capable of massively-parallel analyses of biological or chemical samples are typically limited to laboratory settings because of several factors that can include their large size, lack of portability, requirement of a skilled technician to operate the instrument, power need, need for a controlled operating environment, and cost. When a sample is to be analyzed using such equipment, a common paradigm is to extract a sample at a point of care or in the field, send the sample to the lab and wait for results of the analysis. The wait time for results can range from hours to days.

### SUMMARY OF THE DISCLOSURE

Some aspects of the present disclosure relate to a method for calibrating a system comprising an integrated device, the method comprising: exciting, with light from at least one excitation source, a reference dye molecule disposed in a chamber of the integrated device; obtaining a signal emitted by the reference dye molecule, the signal containing information representative of a bleaching time of the reference dye molecule; and adjusting one or more characteristics of the system based on the bleaching time of the reference dye molecule.

Some aspects of the present disclosure relate to an integrated device comprising: at least one chamber for receiving a reference dye molecule; at least one photodetection region for receiving a signal emitted by the reference dye molecule when excited by light from at least one excitation source, the signal containing information representative of a bleaching time of the reference dye molecule; and at least one controller configured to control adjusting of one or more characteristics of a system comprising the integrated device based on the bleaching time of the reference dye molecule.

Some aspects of the present disclosure relate to a method of manufacturing an integrated device, comprising: providing a chamber on a substrate of the integrated device, the chamber being configured for receiving a reference dye molecule and positioned on the substrate such that the reference dye molecule receives light from at least one light source; providing a photodetection region positioned adjacent to the chamber such that the photodetection region receives a signal emitted by the reference dye molecule when the light from the at least one light source is delivered to the reference dye molecule; and coupling at least one controller to the photodetection region so that the at least one controller receives information contained by the signal emitted by the reference dye molecule, the information being representative of a bleaching time of the reference dye molecule.

Some aspects of the present disclosure relate to a method for determining a measure of quantitative loading of a sample in an integrated device, the method comprising exciting, with excitation light from at least one excitation source, one or more reference dye molecules that, during the exciting with the excitation light, are attached to respective biomolecules of the sample bound to a surface of a chamber of one or more chambers of the integrated device, obtaining a signal emitted by the one or more reference dye molecules in response to the excitation light, and determining, based on the signal emitted by the one or more reference dye molecules, the measure of quantitative loading of the sample.

Some aspects of the present disclosure relate to an integrated device configured to determine a measure of quantitative loading of a sample, the integrated device comprising: at least one chamber for receiving one or more reference dye molecules that, during excitation of the one or more reference dye molecules with excitation light delivered from at least one excitation source, are attached to respective biomolecules of the sample, the respective biomolecules being bound to a surface of the at least one chamber, at least one photodetection region for receiving a signal emitted by the one or more reference dye molecules in response to the excitation light from the at least one excitation source, and at least one controller configured to determine, based on the signal emitted by the one or more reference dye molecules, the measure of quantitative loading of the sample.

Some aspects of the present disclosure relate to a method for determining a measure of quantitative loading of a sample in an integrated device, the method comprising: exciting, with light from at least one excitation source, one or more reference dye molecules, the one or more reference dye molecules being attached to respective secondary biomolecules which reversibly binds to respective biomolecules of the sample, the respective biomolecules being bound to a surface of a chamber of a plurality of chambers of the integrated device, obtaining a signal emitted by the one or more reference dye molecules in response to the excitation light, determining a pulsing pattern of the one or more reference dye molecules, and determining the measure of quantitative loading of the sample based on the pulsing pattern of the one or more reference dye molecules.

### BRIEF DESCRIPTION OF DRAWINGS

The features and advantages of the present disclosure will become more apparent from the detailed description set forth below when taken in conjunction with the drawings. When describing embodiments in reference to the drawings, directional references ("above," "below," "top," "bottom," "left," "right," "horizontal," "vertical," *etc*.) may be used. Such references are intended merely as an aid to the reader viewing the drawings in a normal orientation. These directional references are not intended to describe a preferred or only orientation of features of an embodied device. A device may be embodied using other orientations.

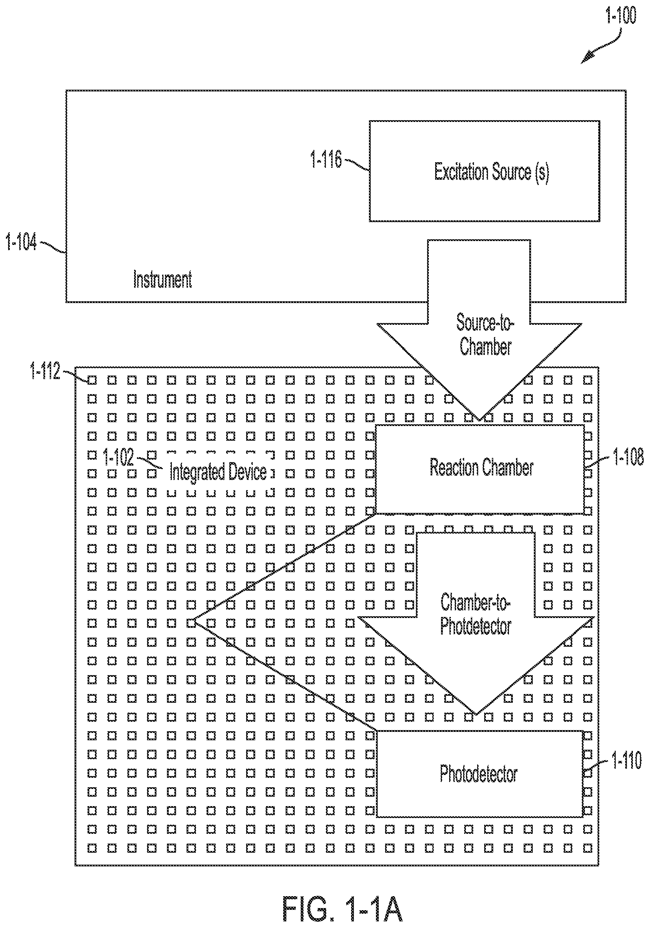

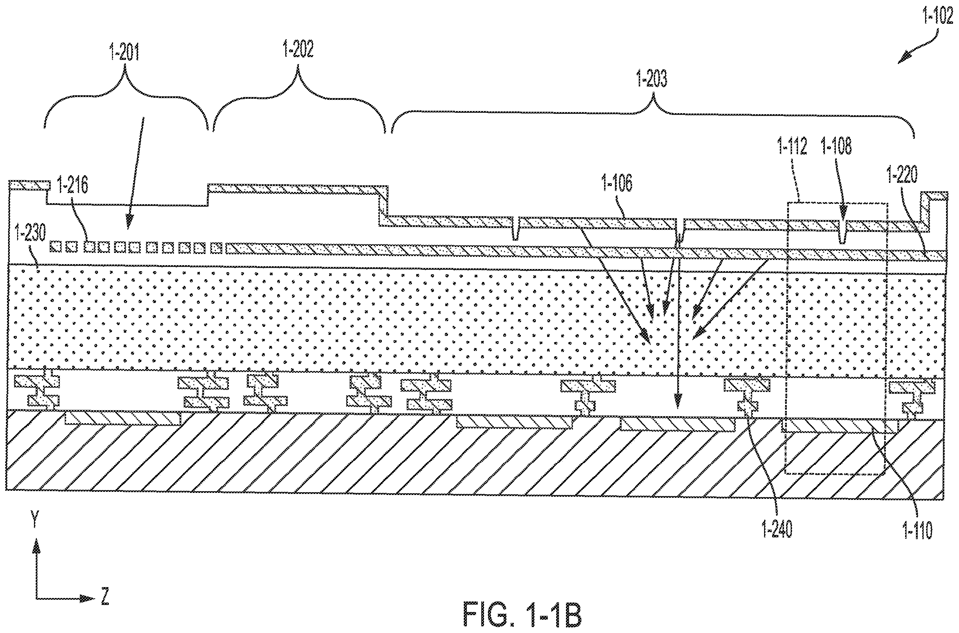

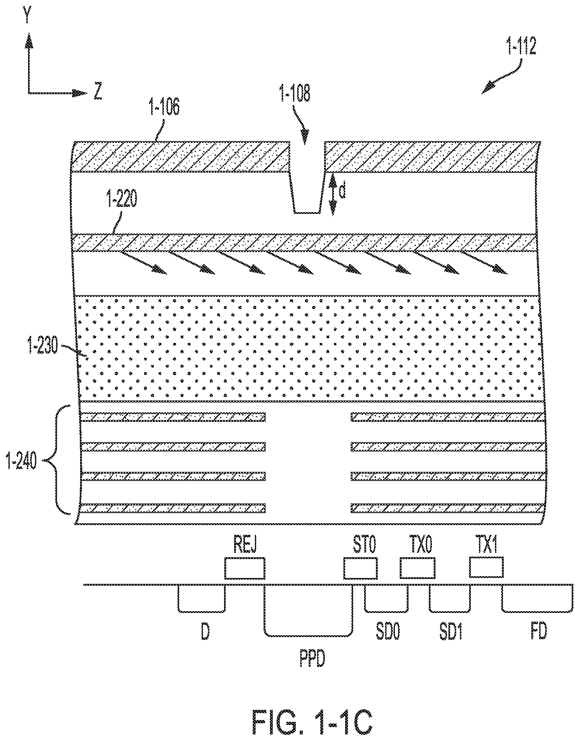

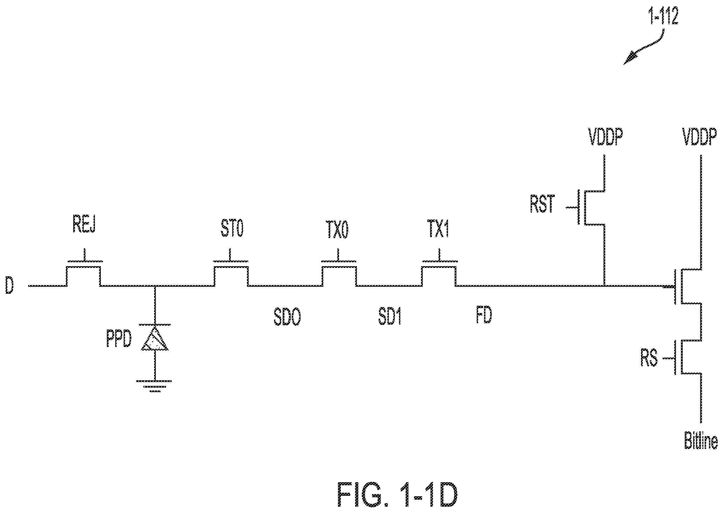

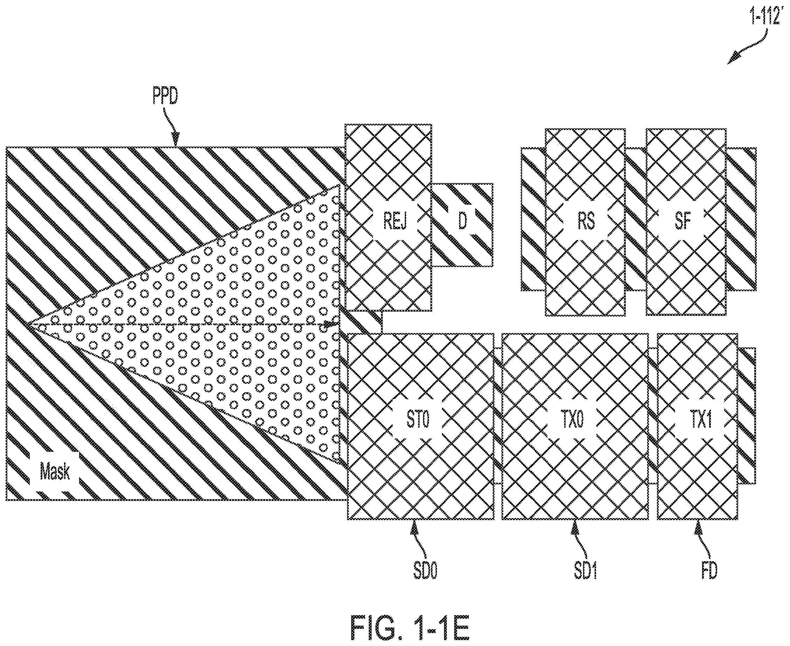

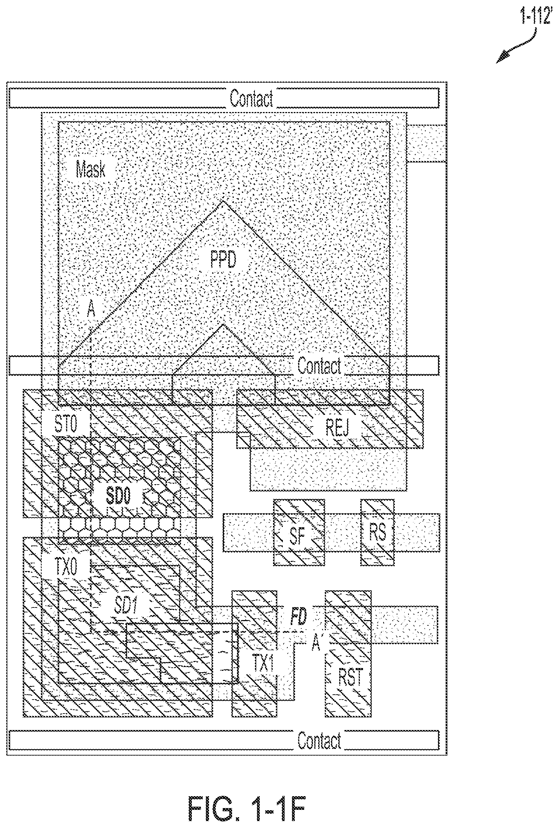

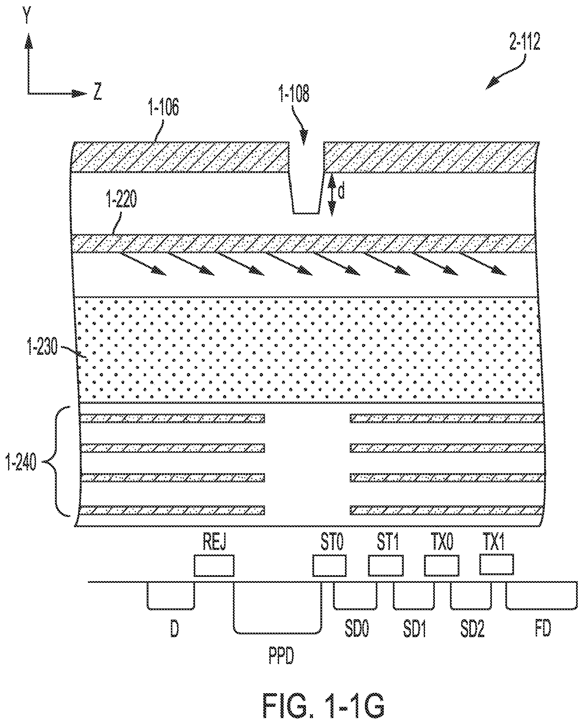

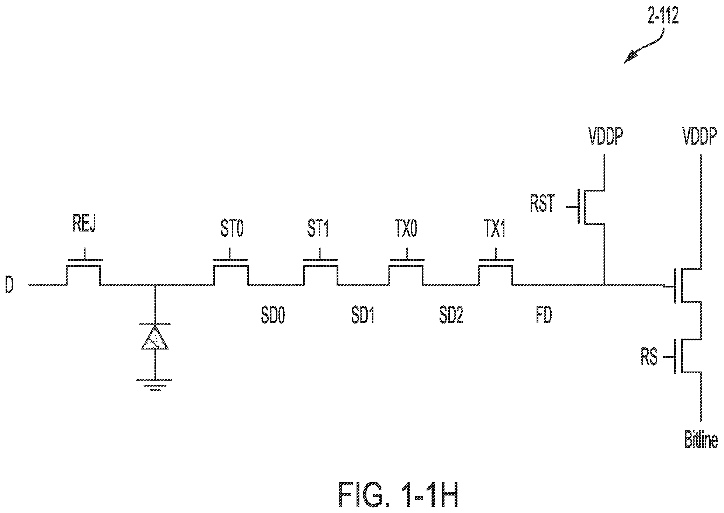

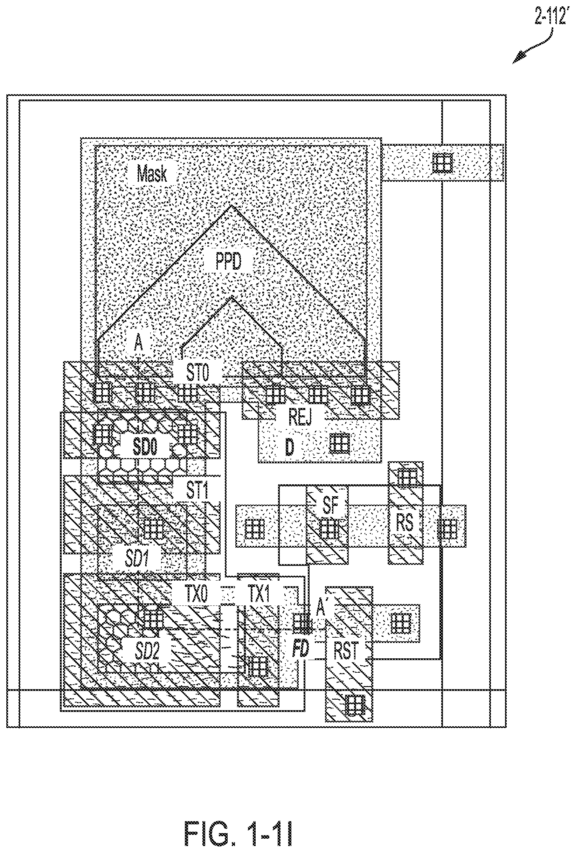

FIG. 1-1A is a block diagram of an integrated device and an instrument, according to some embodiments.
FIG. 1-1B is a schematic of an integrated device, according to some embodiments.
FIG. 1-1C is a schematic of a pixel of an integrated device, according to some embodiments.
FIG. 1-1D is a circuit diagram of the pixel of FIG. 1-1C, according to some embodiments.
FIG. 1-1E is a top view of the pixel of FIG. 1-1C, according to some embodiments.
FIG. 1-1F is a plan view of the pixel of FIGS. 1-1C and 1-1D, according to some embodiments.
FIG. 1-1G is a schematic of an alternative pixel of an integrated device, according to some embodiments.
FIG. 1-1H is a circuit diagram of the pixel of FIG. 1-1G, according to some embodiments.
FIG. 1-1I is a plan view of the pixel of FIGS. 1-1C and 1-1D, according to some embodiments.
FIG. 2 illustrates an example process for calibrating a system comprising an integrated device using photobleaching information, according to some embodiments.
FIG. 3-1 illustrates a dye-labeled sample attached to a surface, according to some embodiments.
FIG. 3-2 illustrates a dye-labeled sample attached in a reaction chamber, according to some embodiments.
FIG. 4 illustrates an example graph illustrating light source excitation power over time, according to some embodiments.
FIG. 5-1 illustrates an example graph illustrating measured signal from a reference dye over time, according to some embodiments.
FIG. 5-2 illustrates example histograms of measured bleaching time and dye intensity for a collection of single molecules in reaction chambers, according to some embodiments.
FIG. 6 illustrates a sequencing reaction in a reaction chamber, according to some embodiments.
FIG. 7 illustrates an example process for quantifying loading of one or more reaction chambers, according to some embodiments.
FIG. 8-1 illustrates an example trace from a chip loading process that represents a single loaded well, according to some embodiments.
FIG. 8-2 illustrates an example trace from a chip loading process that represents a double loaded well, according to some embodiments.
FIG. 8-3 illustrates an example trace from a chip loading process that represents a multi-loaded well, according to some embodiments.
FIG. 9 illustrates a periodic pulsing pattern of fluorescent molecules reversibly bound to a biomolecule, according to some embodiments.
FIG. 10 illustrates an example heatmap of reaction chambers illustrating percent loading, according to some embodiments.

### DETAILED DESCRIPTION

### I. Introduction

Aspects of the present disclosure relate to techniques for calibrating a system comprising an integrated device. According to some embodiments, the calibration techniques use photobleaching information to calibrate aspects of the integrated device and/or components that interact with the integrated device (e.g., laser power, chip configuration, etc.).

Instruments performing analyses of biological or chemical samples by fluorescent emission may be susceptible to photodamage. For example, a light source delivering excitation light to reaction chambers of the integrated device may provide excitation light which is too powerful for performing signal collection and analysis with the integrated device. Photobleaching of a dye occurs when the dye is photochemically altered (e.g., after being exposed to high power laser light for a period of time) such that it is no longer able to fluoresce. High power excitation light may cause fluorescent molecules in reaction chambers of the integrated device to bleach more quickly, which may reduce the amount of useful information that can be obtained from emissions of the fluorescent molecules. The inventors have recognized that calibration techniques applied to an integrated device may prevent photodamage from otherwise occurring.

For example, the inventors have recognized that bleaching information (e.g., bleaching time of a reference molecule) may be used to calibrate a system comprising the integrated device to prevent the photodamage described herein. For example, in some embodiments, a calibration process may be performed prior to performing any sampling and analysis with the integrated device. The calibration process may collect bleaching information about a reference dyes loaded into the reaction chambers by illuminating the reaction chambers with excitation light. The bleaching information may be used to determine a reference excitation intensity for molecules in each reaction chamber which may be used to determine whether to adjust aspects of the integrated device and/or component that interact with the integrated device, including laser power and/or chip configuration.

The inventors have further recognized that differences between the reaction chambers of an integrated device may result in factors, including excitation intensity, to vary between the different reaction chambers of the integrated device. Thus, in some embodiments, a same reference dye is loaded into each of the reaction chambers of the integrated device and differences in excitation intensity between the reaction chambers may be evaluated. Adjustments may be made to the integrated device, for example, to the power of a light source delivering excitation light to the reaction chambers, based on the determined differences in excitation intensity between the reaction chamber to ensure that no reaction chamber receives excitation light that could cause photodamage to the integrated device.

Accordingly, some aspects relate to a method for calibrating a system comprising an integrated device, the method comprising: exciting, with light from at least one excitation source (e.g., a laser), a reference dye molecule disposed in a chamber of the integrated device; obtaining a signal emitted by the reference dye molecule, the signal containing information representative of a bleaching time of the reference dye molecule; and adjusting one or more characteristics (e.g., a power of the at least one excitation source) of the system based on the bleaching time of the reference dye molecule.

In some embodiments, the adjusting comprises decreasing the power of the at least one excitation source when the bleaching time of the reference dye molecule is less than a threshold time. In some embodiments, exciting the reference dye molecule comprises delivering light to the chamber of the integrated device at least until the reference dye molecule undergoes photobleaching.

In some embodiments, the method further comprising determining the bleaching time of the reference dye molecule based on the signal emitted by the reference dye molecule, for example, by determining a duration in which an intensity of the signal emitted by the reference dye molecule exceeds a threshold intensity.

In some embodiments, the method further comprises, subsequent to adjusting the one or more characteristics of the integrated device, operating the integrated device to obtain a signal emitted by a sample disposed in the chamber of the integrated device, wherein the signal emitted by the sample comprises information representative of at least one characteristic (e.g., signal intensity, fluorescence wavelength, fluorescence lifetime, pulse duration and/or interpulse duration) of the sample. In some embodiments, operating the integrated device comprises exciting, with light from the at least one excitation source, the sample such that the sample emits at least one photon; and collecting the at least one photon emitted by the sample with a photodetection region of the integrated device.

In some embodiments, the method further comprises identifying the sample based on the at least one characteristic of the sample. In some embodiments the sample comprises a polypeptide (e.g., a protein and/or a nucleic acid strand such as deoxyribonucleic acid and/or ribonucleic acid). Identifying the sample comprises may comprise identifying one or more amino acids of the sample (e.g., at least 5 amino acids, no more than 50 amino acids, no more than a portion of the polypeptide) based at least in part on the information representative of the at least one characteristic of the sample, and identifying the polypeptide based at least in part on the one or more amino acids. In some embodiments, identifying the sample comprises identifying one or more nucleotides of the DNA and/or RNA strand based at least in part on the information representative of the at least one characteristic of the sample.

In some embodiments, the reference dye molecule comprises a fluorescent molecule immobilized in the chamber of the integrated device.

In some embodiments, the chamber comprises a plurality of chambers; the reference dye molecule comprises a plurality of reference dye molecules disposed in respective ones of the plurality of chambers; and obtaining a signal emitted by the reference dye molecule comprises obtaining a plurality of signals emitted by the plurality of reference dye molecules; and adjusting the one or more characteristics of the integrated device is based on information representative of bleaching times of the plurality of reference dye molecules. In some embodiments, the plurality of reference dye molecules comprises a same molecule.

Some aspects of the present disclosure relate to an integrated device comprising: at least one chamber for receiving a reference dye molecule; at least one photodetection region for receiving a signal emitted by the reference dye molecule when excited by light from at least one excitation source, the signal containing information representative of a bleaching time of the reference dye molecule; and at least one controller configured to control adjusting of one or more characteristics of a system comprising the integrated device based on the bleaching time of the reference dye molecule. In some embodiments, the at least one controller of the integrated device is configured to perform, at least in part, the methods described herein.

Some aspects of the present disclosure relate to a method of manufacturing an integrated device, comprising: providing a chamber on a substrate of the integrated device, the chamber being configured for receiving a reference dye molecule and positioned on the substrate such that the reference dye molecule receives light from at least one light source; providing a photodetection region positioned adjacent to the chamber such that the photodetection region receives a signal emitted by the reference dye molecule when the light from the at least one light source is delivered to the reference dye molecule; and coupling at least one controller to the photodetection region so that the at least one controller receives information contained by the signal emitted by the reference dye molecule, the information being representative of a bleaching time of the reference dye molecule.

Further aspects of the present disclosure further relate to techniques for determining a measure of quantitative loading of an integrated device. For example, an integrated device may comprise a plurality of reaction chambers, as described herein. A sample processed by the integrated device may comprise a plurality of biomolecules (e.g., peptides, nucleic acids). When loaded, each reaction chamber may contain either no biomolecules (empty), a single biomolecule (singly loaded), two biomolecules (doubly loaded), or more than two biomolecules (multi-loaded).

The inventors have recognized that it would be advantageous to obtain a measure of how the reaction chambers are loaded. In particular, the measure may indicate whether a particular reaction chamber is empty, singly loaded, doubly loaded, or multi-loaded. In some embodiments, the measure may indicate a percentage loaded of the entire integrated device.

The quantitative loading information may be determined based on signals emitted from one or more reference dye molecules in response to excitation light delivered from at least one excitation source. For example, in some embodiments, a respective reference dye molecule may be attached to each respective biomolecule present in a reaction chamber. By determining a number of reference dye molecules present in a reaction chamber, a number of biomolecules present in the reaction chamber may be inferred.

In some embodiments, the reference dye molecule may be photobleached, and a number of photobleaching steps may be used to determine the number of reference dye molecules present in a reaction chamber. In some embodiments, the reference dye molecule may be excited by excitation light, without photobleaching, and the relative intensity of signals obtained from the reaction chamber may be used to determine the number of reference dye molecules present in a reaction chamber. In some embodiments, the reference dye molecule may be bound to a secondary biomolecule (e.g., an oligonucleotide, an N-terminal amino acid recognizer). The secondary biomolecule may be bound to the biomolecule. In some embodiments, the secondary biomolecule may reversibly bind to a biomolecule. The reference dye molecule may emit a signal only when the reference dye molecule is bound to the biomolecule (e.g., via the secondary biomolecule) and excited by excitation light. A characteristic pulse pattern of the reference dye molecule may be used to determine the number of reference dye molecules present in a reaction chamber.

Such information may be used to optimize use of the integrated device. For example, in some embodiments, the quantitative loading information may be used during the loading process (e.g., to continuously monitor loading of the integrated device). In some embodiments, the quantitative loading information may be used to determine whether to continue loading the integrated device with additional sample (e.g., when it is determined that a large number of wells remain empty). In some embodiments, the quantitative loading information may be used to adjust how loading is performed (e.g., adjusting a concentration of biomolecules in the sample being loaded).

In some embodiments, the quantitative loading information may be used to optimize use of the integrated device after completing the loading step. For example, the quantitative loading information may be used when performing future loadings of the integrated device. In some embodiments, the quantitative loading information may be used to determine which reaction chambers to process signals from.

Accordingly, the inventors have developed techniques for determining a measure of quantitative loading of a sample loaded onto an integrated device. Some aspects of the present disclosure relate to a method for determining a measure of quantitative loading of a sample in an integrated device (e.g., a number of biomolecules present in a single chamber of the one or more chambers, a percentage of the one or more chambers containing a single biomolecule of the sample), the method comprising: exciting, with light from at least one excitation source, one or more reference dye molecules that, during the exciting with the excitation light, are attached to (e.g., covalently, non-covalently) respective biomolecules of the sample (e.g., a peptide, a nucleic acid) bound to a surface of a chamber of one or more chambers of the integrated device; obtaining a signal emitted by the one or more reference dye molecules in response to the excitation light (e.g., a signal expressing an intensity of light emitted by the one or more reference dye molecules over a period of time); and determining, based on the signal emitted by the one or more reference dye molecules, the measure of quantitative loading of the sample.

In some embodiments, at least some of the one or more reference dye molecules are attached to a respective linker to which the respective biomolecule is bound, wherein the respective linker is bound to a base of the chamber.

In some embodiments, exciting the one or more reference dye molecules comprises photobleaching the one or more reference dye molecules. The method may further comprise determining a number of photobleaching steps in the signal emitted by the one or more reference dye molecules and determining a number of biomolecules that are bound to the surface of the chamber based on the number of photobleaching steps.

In some embodiments, the method further comprises optimizing operation of the integrated device based on the measure of quantitative loading. For example, optimizing operation of the integrated device based on the measure of quantitative loading may comprise adjusting how loading of the sample onto the integrated device is performed (e.g., by adjusting a concentration of biomolecules in the sample). In some embodiments, adjusting how loading of the sample is performed comprises adjusting how loading of the sample is performed to maximize a number of the one or more chambers that contain a single biomolecule bound to a surface thereof. In some embodiments, optimizing operation of the integrated device based on the measure of quantitative loading comprises excluding signals from at least some of the one or more chambers from subsequent analysis.

In some embodiments, the method further comprises reloading the one or more chambers with additional sample until an optimal number of the one or more chambers each contain at least one and no more than one biomolecule bound to a surface of a respective one or the one or more chambers (e.g., by using the measure of quantitative loading to determine when the optimal number of the one or more chambers contain the single biomolecule).

In some embodiments, the method further comprises terminating loading when the optimal number of the one or more chambers contain the at least one and no more than one biomolecule of the sample (e.g., by removing unbound reference dye molecules from the integrated device).

In some embodiments, the method further comprises, subsequent to terminating loading, sequencing the sample at least in part by: (1) delivering excitation light to the one or more chambers; (2) obtaining signals emitted from the one or more chambers in response to the excitation light; and (3) identifying one or more biomolecules of the sample based on the signals.

In some embodiments, each of the one or more reference dye molecules, when attached to the respective biomolecule, is separated from the respective biomolecule by at least 1 nm.

In some embodiments, the one or more reference dye molecules are attached to the respective biomolecule via a secondary biomolecule (e.g., an oligonucleotide, an N-terminal amino acid recognizer). That is the secondary biomolecule may be bound to the respective biomolecule, and the reference dye molecule may be bound to the secondary biomolecule.

In some embodiments, the secondary biomolecule may be reversibly bound to the respective biomolecule. For example, each secondary biomolecule may bind to a respective biomolecule periodically. The one or more reference dye molecules may exhibit a pulsing pattern, such that, a respective reference dye molecule of the one or more reference dye molecules, excited by excitation light, emits emission light only when bound to the respective biomolecule (via a respective secondary biomolecule). In some embodiments, the measure of quantitative loading of the sample may comprise a number of biomolecules present in the chamber and the method may further comprise determining the number of biomolecules present in the chamber based on the pulsing pattern of the one or more reference dye molecules.

Some aspects of the present disclosure relate to an integrated device configured to determine a measure of quantitative loading of a sample, the integrated device comprising: at least one chamber for receiving one or more reference dye molecules, the one or more reference dye molecules being attached to respective biomolecules of the sample; at least one photodetection region for receiving a signal emitted by the one or more reference dye molecules, the signal expressing an intensity of light emitted by the one or more reference dye molecules in response to excitation light from at least one excitation source over a period of time; and at least one controller configured to determine, based on the signal emitted by the one or more reference dye molecules, the measure of quantitative loading of the sample.

Some aspects of the present disclosure relate to a method for determining a measure of quantitative loading of a sample in an integrated device, the method comprising: exciting, with light from at least one excitation source, one or more reference dye molecules, the one or more reference dye molecules being attached to respective secondary biomolecules (e.g., oligonucleotides, N-terminal amino acid recognizers) which reversibly binds to respective biomolecules of the sample, the respective biomolecules being bound to a surface of a chamber of a plurality of chambers of the integrated device; obtaining a signal emitted by the one or more reference dye molecules in response to the excitation light; determining a pulsing pattern of the one or more reference dye molecules; and determining the measure of quantitative loading of the sample based on the pulsing pattern of the one or more reference dye molecules.

In some embodiments, the one or more reference dye molecules may bind to the respective biomolecules periodically. In some embodiments, the one or more reference dye molecules may bind to the respective biomolecule once per pulsing period; the pulsing pattern comprises one or more pulses or no pulses; and the measure of quantitative loading is determined based on a number of the one or more pulses received during a pulsing period.

In some embodiments, the measure of quantitative loading comprises a number of biomolecules present in the chamber. In some embodiments, determining the measure of quantitative loading of the sample comprises, when no pulses are present in the pulsing pattern, determining that the chamber contains no biomolecules.

In some embodiments, the one or more reference dye molecules may emit emission light only when bound to the respective biomolecule.

In some embodiments, the measure of quantitative loading of the sample comprises a number of the respective biomolecules of the sample bound to the surface of the chamber. Determining the measure of quantitative loading of the sample may comprise, when the no pulses are present in the pulsing pattern, determining that the chamber contains no biomolecules.

The aspects and embodiments described above, as well as additional aspects and embodiments, are described further below. These aspects and/or embodiments may be used individually, all together, or in any combination, as the application is not limited in this respect.

### II. Integrated Device Overview

As described herein, the techniques described herein may facilitate determining quantitative loading of an integrated device. The integrated device may facilitate providing excitation light from one or more excitation sources located separate from a pixel array of an integrated device to a reaction chamber containing a sample. The excitation light may be directed at least in part by elements of the integrated device towards one or more pixels to illuminate an illumination region within the reaction chamber. A sample disposed in the reaction chamber, or a reaction component attached to the sample (such as a fluorescent label, for example), may emit emission light when located within the illumination region of the reaction chamber and in response to being illuminated by the excitation light. In some embodiments, the one or more excitation sources are part of a system comprising the integrated device.

Emission light emitted from one or more reaction chambers (e.g., at least two reaction chambers, in some embodiments), may be detected by one or more photodetectors within a pixel of the integrated device. As described herein, the integrated device may be configured having multiple pixels (e.g., an array of pixels), and thus, may have multiple reaction chambers and corresponding photodetectors. Characteristics of the detected emission light may provide an indication for identifying the label associated with the emission light. Such characteristics may include any suitable type of characteristic, including an arrival time of photons detected by the photodetector, an amount of photons accumulated over time by a photodetector, and/or a distribution of photons across two or more photodetectors. In some embodiments, a photodetector may have a configuration that allows for detection of one or more characteristics associated with the emission light, such as timing characteristics (e.g., fluorescence lifetime, pulse duration, interpulse duration), wavelength, and/or intensity. As one example, one or more photodetectors may detect a distribution of photon arrival times after a pulse of excitation light propagates through the integrated device, and the distribution of arrival times may provide an indication of a timing characteristic of the emission light (e.g., a proxy for fluorescence lifetime, pulse duration, and/or interpulse duration). Such information may be used in techniques for detection and/or identification of molecules in a sample, for example, including those described in U.S. Pat. Application No. 16/686,028 titled "METHODS AND COMPOSITIONS FOR PROTEIN SEQUENCING," filed November 15, 2019 under Attorney Docket No. R0708.70042US02, PCT Application No. PCT/US19/61831 titled "METHODS AND COMPOSITIONS FOR PROTEIN SEQUENCING," filed November 15, 2019 under Attorney Docket No. R0708.70042WO00, U.S. Pat. Application No. 62/984,229 titled "INTEGRATED SENSOR FOR MULTI-DIMENSIONAL SIGNAL ANALYSIS," filed March 2, 2020 under Attorney Docket No. R0708.70090US00, U.S. Pat. Application No. 15/600,979 titled "LABELED NUCLEOTIDE COMBINATIONS AND METHODS FOR NUCLEIC ACID SEQUENCING," filed May 22, 2017 under Attorney Docket No. R0708.70018US02, and U.S. Pat. Application No. 15/161,125 titled "METHODS FOR NUCLEIC ACID SEQUENCING," filed May 20, 2016 under Attorney Docket No. R0708.70020US00 each of which are incorporated by reference in their entireties. In some embodiments, one or more photodetectors provide an indication of a probability of emission light emitted by the fluorescent labels (e.g., fluorescence intensity). In some embodiments, one or more photodetectors may be sized and arranged to capture a spatial distribution of the emission light (e.g., wavelength). Output signals from the one or more photodetectors may be used to distinguish a fluorescent label from among a plurality of labels, where the plurality of labels may be used to identify a sample or it structure, as described herein.

For example, a schematic overview of an exemplary system 1-100 is illustrated in FIG. 1-1A. The system comprises both an integrated device 1-102 that interfaces with an instrument 1-104. In some embodiments, instrument 1-104 may include one or more excitation sources 1-116 integrated as part of instrument 1-104. In some embodiments, an excitation source may be external to both instrument 1-104 and integrated device 1-102, and instrument 1-104 may be configured to receive excitation light from the excitation source and direct excitation light to the integrated device. The integrated device may interface with the instrument using any suitable socket for receiving the integrated device and holding it in precise optical alignment with the excitation source. The excitation source 1-116 may be configured to provide excitation light to the integrated device 1-102. As illustrated schematically in FIG. 1-1A, the integrated device 1-102 has a plurality of pixels 1-112, where at least a portion of pixels may perform independent analysis of a sample of interest. Such pixels 1-112 may be referred to as "passive source pixels" since a pixel receives excitation light from an excitation source 1-116 separate from the pixel, where excitation light from the source excites some or all of the pixels 1-112. Excitation source 1-116 may be any suitable light source. Examples of suitable excitation sources are described in U.S. Pat. Application No. 14/821,688, filed August 7, 2015, titled "INTEGRATED DEVICE FOR PROBING, DETECTING AND ANALYZING MOLECULES" under Attorney Docket Number R0708.70004US02, which is incorporated by reference in its entirety. In some embodiments, excitation source 1-116 includes multiple excitation sources that are combined to deliver excitation light to integrated device 1-102. The multiple excitation sources may be configured to produce multiple excitation energies or wavelengths.

Referring to FIG. 1-1B, a pixel 1-112 has a reaction chamber 1-108 configured to receive a at least one sample of interest and a photodetector 1-110 for detecting emission light emitted from the reaction chamber in response to illuminating the sample and at least a portion of the reaction chamber 1-108 with excitation light provided by the excitation source 1-116. In some embodiments, reaction chamber 1-108 may retain the sample in proximity to a surface of integrated device 1-102, which may ease delivery of excitation light to the sample and detection of emission light from the sample or a reaction component (e.g., a fluorescent label). As shown in the illustrated embodiment of FIG. 1-1B, the reaction chamber 1-108 and the photodetector 1-110 have a one-to-one correspondence. In some embodiments, as described herein, each pixel may comprise multiple reaction chambers per photodetector.

Optical elements for coupling excitation light from excitation light source 1-116 to integrated device 1-102 and guiding excitation light to the reaction chamber 1-108 may be located on one or both of the integrated device 1-102 and the instrument 1-104. Source-to-chamber optical elements may comprise one or more grating couplers located on integrated device 1-102 to couple excitation light to the integrated device and waveguides to deliver excitation light from instrument 1-104 to reaction chambers in pixels 1-112. One or more optical splitter elements may be positioned between a grating coupler and the waveguides. The optical splitter may couple excitation light from the grating coupler and deliver excitation light to at least one of the waveguides. In some embodiments, the optical splitter may have a configuration that allows for delivery of excitation light to be substantially uniform across all the waveguides such that each of the waveguides receives a substantially similar amount of excitation light. Such embodiments may improve performance of the integrated device by improving the uniformity of excitation light received by reaction chambers of the integrated device.

Reaction chamber 1-108, a portion of the excitation source-to-chamber optics, and the reaction chamber-to-photodetector optics are located on integrated device 1-102. Excitation source 1-116 and a portion of the source-to-chamber components are located in instrument 1-104. In some embodiments, a single component may play a role in both coupling excitation light to reaction chamber 1-108 and delivering emission light from reaction chamber 1-108 to photodetector 1-110. Examples of suitable components, for coupling excitation light to a reaction chamber and/or directing emission light to a photodetector, to include in an integrated device are described in U.S. Pat. Application No. 14/821,688, filed August 7, 2015, titled "INTEGRATED DEVICE FOR PROBING, DETECTING AND ANALYZING MOLECULES" under Attorney Docket Number R0708.70004US02 and U.S. Pat. Application No. 14/543,865, filed November 17, 2014, titled "INTEGRATED DEVICE WITH EXTERNAL LIGHT SOURCE FOR PROBING, DETECTING, AND ANALYZING MOLECULES" under Attorney Docket Number R0708.70005US00, both of which are incorporated by reference in their entirety.

Pixel 1-112 is associated with its own individual reaction chamber 1-108 and at least one photodetector 1-110. The plurality of pixels of integrated device 1-102 may be arranged to have any suitable shape, size, and/or dimensions. Integrated device 1-102 may have any suitable number of pixels. The number of pixels in integrated device 1-102 may be in the range of approximately 100,000 pixels to 64,000,000 pixels or any value or range of values within that range. In some embodiments, the pixels may be arranged in an array of 1024 pixels by 2048 pixels. Integrated device 1-102 may interface with instrument 1-104 in any suitable manner. In some embodiments, instrument 1-104 may have an interface that detachably couples to integrated device 1-102 such that a user may attach integrated device 1-102 to instrument 1-104 for use of integrated device 1-102 to analyze at least one sample of interest in a suspension and remove integrated device 1-102 from instrument 1-104 to allow for another integrated device to be attached. The interface of instrument 1-104 may position integrated device 1-102 to couple with circuitry of instrument 1-104 to allow for readout signals from one or more photodetectors to be transmitted to instrument 1-104. Integrated device 1-102 and instrument 1-104 may include multi-channel, high-speed communication links for handling data associated with large pixel arrays (e.g., more than 10,000 pixels).

Instrument 1-104 may include a user interface for controlling operation of instrument 1-104 and/or integrated device 1-102. The user interface may be configured to allow a user to input information into the instrument, such as commands and/or settings used to control the functioning of the instrument. In some embodiments, the user interface may include buttons, switches, dials, and a microphone for voice commands. The user interface may allow a user to receive feedback on the performance of the instrument and/or integrated device, such as proper alignment and/or information obtained by readout signals from the photodetectors on the integrated device. In some embodiments, the user interface may provide feedback using a speaker to provide audible feedback. In some embodiments, the user interface may include indicator lights and/or a display screen for providing visual feedback to a user.

In some embodiments, instrument 1-104 may include a computer interface configured to connect with a computing device. Computer interface may be a USB interface, a FireWire interface, or any other suitable computer interface. Computing device may be any general purpose computer, such as a laptop or desktop computer. In some embodiments, computing device may be a server (e.g., cloud-based server) accessible over a wireless network via a suitable computer interface. The computer interface may facilitate communication of information between instrument 1-104 and the computing device. Input information for controlling and/or configuring the instrument 1-104 may be provided to the computing device and transmitted to instrument 1-104 via the computer interface. Output information generated by instrument 5-104 may be received by the computing device via the computer interface. Output information may include feedback about performance of instrument 1-104, performance of integrated device 1-102, and/or data generated from the readout signals of photodetector 1-110.

In some embodiments, instrument 1-104 may include a processing device configured to analyze data received from one or more photodetectors of integrated device 1-102 and/or transmit control signals to excitation source(s) 1-116. In some embodiments, the processing device may comprise a general purpose processor, a specially-adapted processor (e.g., a central processing unit (CPU) such as one or more microprocessor or microcontroller cores, a field-programmable gate array (FPGA), an application-specific integrated circuit (ASIC), a custom integrated circuit, a digital signal processor (DSP), or a combination thereof.) In some embodiments, the processing of data from one or more photodetectors may be performed by both a processing device of instrument 1-104 and an external computing device. In other embodiments, an external computing device may be omitted and processing of data from one or more photodetectors may be performed solely by a processing device of integrated device 1-102.

A cross-sectional schematic of integrated device 1-102 illustrating a row of pixels 1-112 is shown in FIG. 1-1B. Integrated device 1-102 may include coupling region 1-201, routing region 1-202, and pixel region 1-203. Pixel region 1-203 may include a plurality of pixels 1-112 having reaction chambers 1-108 positioned on a surface at a location separate from coupling region 1-201, which is where excitation light (shown as the dashed arrow) couples to integrated device 1-102. Reaction chambers 1-108 may be formed through metal layer(s) 1-106. One pixel 1-112, illustrated by the dotted rectangle, is a region of integrated device 1-102 that includes a reaction chamber 1-108 and a photodetection region having one or more photodetectors 1-110. In the illustrated embodiment, the pixel comprises a single reaction chamber 1-108. In some embodiments, each pixel may comprise two or more reaction chambers.

FIG. 1-1B illustrates the path of excitation (shown in dashed lines) by coupling a beam of excitation light to coupling region 1-201 and to reaction chambers 1-108. The row of reaction chambers 1-108 shown in FIG. 1-1B may be positioned to optically couple with waveguide 1-220. Excitation light may illuminate a sample located within a reaction chamber. The sample or reaction component (e.g., a fluorescent label) may reach an excited state in response to being illuminated by the excitation light. When a sample or reaction component is in an excited state, the sample or reaction component may emit emission light, which may be detected by one or more photodetectors associated with the reaction chamber. FIG. 1-1B schematically illustrates an optical axis of emission light (shown as the solid line) from a reaction chamber 1-108 to photodetector(s) 1-110 of pixel 1-112. The photodetector(s) 1-110 of pixel 1-112 may be configured and positioned to detect emission light from reaction chamber 1-108. Examples of suitable photodetectors are described in U.S. Pat. Application 14/821,656, filed August 7, 2015, titled "INTEGRATED DEVICE FOR TEMPORAL BINNING OF RECEIVED PHOTONS" under Attorney Docket Number R0708.70002US02, which is incorporated by reference in its entirety. For an individual pixel 1-112, a reaction chamber 1-108 and its respective photodetector(s) 1-110 may be aligned along a common axis (along the y-direction shown in FIG. 1-1A). In this manner, the photodetector(s) may overlap with the reaction chamber within a pixel 1-112.

The directionality of the emission light from a reaction chamber 1-108 may depend on the positioning of the sample in the reaction chamber 1-108 relative to metal layer(s) 1-106 because metal layer(s) 1-106 may act to reflect emission light. In this manner, a distance between metal layer(s) 1-106 and a fluorescent marker positioned in a reaction chamber 1-108 may impact the efficiency of photodetector(s) 1-110, that are in the same pixel as the reaction chamber, to detect the light emitted by the fluorescent marker. The distance between metal layer(s) 1-106 and the bottom surface of a reaction chamber 1-108, which is proximate to where a sample may be positioned during operation, may be in the range of 100 nm to 500 nm, or any value or range of values in that range. In some embodiments the distance between metal layer(s) 1-106 and the bottom surface of a reaction chamber 1-108 is approximately 300 nm.

The distance between the sample and the photodetector(s) may also impact efficiency in detecting emission light. By decreasing the distance light has to travel between the sample and the photodetector(s), detection efficiency of emission light may be improved. In addition, smaller distances between the sample and the photodetector(s) may allow for pixels that occupy a smaller area footprint of the integrated device, which can allow for a higher number of pixels to be included in the integrated device. The distance between the bottom surface of a reaction chamber 1-108 and photodetector(s) may be in the range of 1 µm to 15 µm, or any value or range of values in that range. It should be appreciated that, in some embodiments, emission light may be provided through other means than an excitation light source and a reaction chamber. Accordingly, some embodiments may not include reaction chamber 1-108.

Photonic structure(s) 1-230 may be positioned between reaction chambers 1-108 and photodetectors 1-110 and configured to reduce or prevent excitation light from reaching photodetectors 1-110, which may otherwise contribute to signal noise in detecting emission light. As shown in FIG. 1-1B, the one or more photonic structures 1-230 may be positioned between waveguide 1-220 and photodetectors 1-110. Photonic structure(s) 1-230 may include one or more optical rejection photonic structures including a spectral filter, a polarization filter, and a spatial filter. Photonic structure(s) 1-230 may be positioned to align with individual reaction chambers 1-108 and their respective photodetector(s) 1-110 along a common axis. Metal layers 1-240, which may act as a circuitry for integrated device 1-102, may also act as a spatial filter, or polarization filter, in accordance with some embodiments. In such embodiments, one or more metal layers 1-240 may be positioned to block some or all excitation light from reaching photodetector(s) 1-110.

Coupling region 1-201 may include one or more optical components configured to couple excitation light from an external excitation source, for example, excitation source(s) 1-116 illustrated in FIG. 1-1A. Coupling region 1-201 may include grating coupler 1-216 positioned to receive some or all of a beam of excitation light. Examples of suitable grating couplers are described in U.S. Pat. Application 15/855,403, filed December 15, 2017, titled "OPTICAL COUPLER AND WAVEGUIDE SYSTEM" under Attorney Docket Number R0708.70021US01, and U.S. Pat. Application 16/861,399, filed April 29, 2020, titled "SLICED GRATING COUPLER WITH INCREASED BEAM ALIGNMENT SENSITIVITY" under Attorney Docket Number R0708.70071US01, each of which are hereby incorporated by reference herein in their entireties. Grating coupler 1-216 may couple excitation light to waveguide 1-220, which may be configured to propagate excitation light to the proximity of one or more reaction chambers 1-108. Alternatively, coupling region 1-201 may comprise other well-known structures for coupling light into a waveguide.

Components located off of the integrated device may be used to position and align the excitation source 1-116 to the integrated device. Such components may include optical components including lenses, mirrors, prisms, windows, apertures, attenuators, and/or optical fibers. Additional mechanical components may be included in the instrument to allow for control of one or more alignment components. Such mechanical components may include actuators, stepper motors, and/or knobs. Examples of suitable excitation sources and alignment mechanisms are described in U.S. Pat. Application 15/161,088, filed May 20, 2016, titled "PULSED LASER AND SYSTEM" under Attorney Docket Number R0708.70010US02, which is incorporated by reference in its entirety. Another example of a beam-steering module is described in U.S. Pat. Application 15/842,7720, filed December 14, 2017, titled "COMPACT BEAM SHAPING AND STEERING ASSEMBLY" under Attorney Docket Number R0708.70024US01, which is incorporated herein by reference.

A sample to be analyzed may be introduced into reaction chamber 1-108 of pixel 1-112. The sample may be a biological sample or any other suitable sample, such as a chemical sample. The sample may include multiple molecules and the reaction chamber may be configured to isolate a single molecule. In some instances, the dimensions of the reaction chamber may act to confine a single molecule within the reaction chamber, allowing measurements to be performed on the single molecule. Excitation light may be delivered into the reaction chamber 1-108, so as to excite the sample or at least one fluorescent marker attached to the sample or otherwise associated with the sample while it is within an illumination area within the reaction chamber 1-108.

In operation, parallel analyses of samples within the reaction chambers are carried out by exciting some or all of the samples within the wells using excitation light and detecting signals from sample emission with the photodetectors. Emission light from a sample may be detected by a corresponding photodetector and converted to at least one electrical signal. Information regarding various characteristics of the emission light (e.g., wavelength, fluorescence lifetime, intensity, pulse duration and/or any other suitable characteristic) may be collected and used for subsequent analysis, as described herein. The electrical signals may be transmitted along conducting lines (*e*.*g*., metal layers 1-240) in the circuitry of the integrated device, which may be connected to an instrument interfaced with the integrated device. The electrical signals may be subsequently processed and/or analyzed. Processing or analyzing of electrical signals may occur on a suitable computing device either located on or off the instrument.

FIG. 1-1C illustrates a cross-sectional view of a pixel 1-112 of integrated device 1-102, according to some embodiments. FIG. 1-1D shows a circuit diagram of pixel 1-112. FIG. 1 sh-1E shows an exemplary array of pixels 1-112 and processing circuit 1-114, which may be included in integrated device 1-102, according to some embodiments.

In FIGS. 1-1C and 1-1D, pixel 1-112 includes a photodetection region, which may be a pinned photodiode (PPD), two charge storage regions, which may be storage diodes (SD0 and SD1), and a readout region, which may be a floating diffusion (FD) region. Also as shown, pixel 1-112 also includes drain region D and transfer gates ST0, TX0, TX1, and REJ.

In some embodiments, photodetection region PPD, charge storage regions SD0 and SD1 and readout region FD may be formed on an integrated circuit substrate by doping parts of the substrate. For example, the substrate may be lightly doped and photodetection region PPD, charge storage regions SD0 and SD1, and readout region FD may be more heavily doped. In this example, the substrate may be lightly p-type doped and photodetection region PPD, charge storage regions SD0 and SD1, and readout region FD may be n-type doped. Alternatively, the substrate may be lightly n-type doped and photodetection region PPD, charge storage regions SD0 and SD1, and readout region FD may be p-type doped, as embodiments described herein are not so limited.

In some embodiments photodetection region PPD may be configured to generate charge carriers (e.g., photo-electrons) when incident photons are received therein. In some embodiments, charge storage regions SD0 and SD1 may be electrically coupled to photodetection region PPD and/or to one another. For example, pixel 1-112 may include one or more transfer channels electrically coupling charge storage regions SD0 and SD1 to photodetection region PPD and/or to one another. In some embodiments, the transfer channels may be formed by doping portions of the integrated circuit substrate disposed between the regions. For example, the portions may be doped with a same conductivity type as the regions (e.g., an n-type doped channel disposed between an n-type doped PPD and SD0). Referring to FIG. 1-1D, for example, a channel of a transistor coupled between photodetection region PPD and charge storage region SD0 is a transfer channel electrically coupling photodetection region PPD to charge storage region SD0. Similarly, a channel of a transistor coupled between charge storage regions SD0 and SD1 is a transfer channel electrically coupling charge storage region SD0 to SD1, a channel of a transistor coupled between charge storage region SD1 and readout region FD is a transfer channel electrically coupling charge storage region SD1 to readout region FD. A channel of a transistor coupled between photodetection region PPD and drain region D is a transfer channel between photodetection region PPD and drain region D.

In some embodiments, transfer gates ST0, TX0, TX1, and REJ may be configured to control the transfer of charge carriers from photodetection region PPD to storage regions SD0 and SD1, between charge storage regions SD0 and SD1, and/or between charge storage regions SD0 and SD1 and readout region FD. For example, transfer gates ST0, TX0, TX1, and REJ may be electrically coupled to and configured to bias the transfer channels electrically coupling the regions of pixel 1-112 to transfer the charge carriers between the regions when appropriate control signals are applied to the transfer gates. The transfer gates may me conductively (e.g., physically) coupled to the transfer channels, and/or may be positioned close enough to the transfer channels and/or separated by a thin enough insulator to capacitively couple to the transfer channels, according to various embodiments. In some embodiments, transfer gates described herein may be formed using a conductive material such as metal. Alternatively or additionally, in some embodiments, transfer gates described herein may be formed using a semiconductor material such as polysilicon. In some embodiments, materials used to form transfer gates described herein may be at least partially opaque.

In some embodiments, when a control signal is received at a transfer gate, the transfer gate may electrically couple the control signal to the transfer channel and bias the transfer channel, thereby increasing the conductivity of the transfer channel. In some embodiments, the transfer channel may be doped with a same conductivity type but a lower dopant concentration than the regions of pixel 1-112 electrically coupled by the transfer channel, thereby generating an intrinsic electric potential barrier between the regions. The intrinsic electric potential barrier may exist between the regions even when no external electric field is applied to the transfer gate or transfer channel. For example, the dopant concentration of the transfer channel between photodetection region PPD and charge storage region SD0 may generate an intrinsic electric potential barrier between photodetection region PPD and charge storage region SD0. In some embodiments, a control signal may be applied to the transfer gate, the control signal being configured to lower the intrinsic electric potential barrier between the regions electrically coupled by the transfer channel, thereby increasing the conductivity of the transfer channel, and causing a transfer of charge carriers between the regions. For example, for an n-type doped transfer channel, the control signal may have a voltage that is greater than a voltage at one of the regions (e.g., at the source terminal of the transfer channel) by at least a threshold voltage of the transfer channel, the threshold voltage being dependent on the size of the transfer channel, a substrate voltage of the integrated device 1-102 proximate the transfer channel, and other such parameters. Similarly, for a p-type doped transfer channel, the control signal may have a voltage that is lower than the voltage at the one of the regions by at least the threshold voltage. In some embodiments, a control circuit of integrated device 1-102 may be configured to generate and provide such control signals to the transfer gates, as described further herein.

In FIG. 1-1C, pixel 1-112 is shown in a configuration configured to receive incident photons in a direction in which photodetection region PPD, charge storage regions SD0 and SD1, and readout region FD are spaced from transfer gates REJ, ST0, TX0, and TX1 (e.g., front-side illumination). It should be appreciated, however, that in some embodiments, photodetection region PPD may be configured to receive incident photons in a direction in which transfer gates REJ, ST0, TX0, and TX1 are spaced from photodetection region PPD, charge storage regions SD0 and SD1, and readout region FD (e.g., back-side illumination). In some embodiments, such a configuration may improve the electrical characteristics of the transfer gates because the optical characteristics of the transfer gates have a reduced impact on the incident photons.

In FIG. 1-1D, pixel 1-112 further includes a reset (RST) transfer gate coupled to readout region FD and configured for coupling to a high voltage VDDP, and a row select (RS) transfer gate coupled between readout region FD and a bitline. When the integrated device 1-102 is coupled to a power source (e.g., at least a DC power supply), transfer gate RST may be coupled to high voltage VDDP, which is supplied by the power source and/or regulated by a voltage regulator of integrated device 1-102.

In some embodiments, transfer gate RST may be configured to reset a voltage of readout region FD. For example, when a reset signal is applied to transfer gate RST, transfer gate RST may bias the transfer channel electrically coupling readout region FD to high voltage VDDP, thereby increasing the conductivity of the transfer channel and transferring charge carriers from readout region FD to high voltage VDDP. In some embodiments, reset transfer gate RST may be further configured to reset the voltage of charge storage region SD0 and/or SD1. For example, when a reset signal is applied to reset transfer gate RST and a control signal is applied to transfer gate TX1, transfer gate TX1 may transfer charge carriers in charge storage region SD1 to readout region FD and transfer gate RST may transfer the charge carriers to high voltage VDDP. Similarly, when a reset signal is applied to reset transfer gate RST and control signals are applied to transfer gates TX1 and TX0, transfer gate TX0 may transfer charge carriers in charge storage region SD0 to SD1, transfer gate TX1 may transfer the charge carriers in charge storage region SD1 to readout region FD, and transfer gate RST may transfer the charge carriers to high voltage VDDP. In some embodiments, integrated device 1-102 may be configured to reset readout region FD and charge storage regions SD0 and SD1 before collecting and reading out charge carriers. For example, integrated device 1-102 may be configured to reset readout region FD, then reset charge storage region SD1, and then reset charge storage region SD0, before collecting and reading out charge carriers.

In some embodiments, the bitline may be coupled to processing circuitry on the integrated device 1-102 and/or an external circuit configured to receive a voltage level indicative of charge carriers read out to readout region FD. In some embodiments, processing circuitry 1-114 may include an analog-to-digital converter (ADC). In some embodiments, integrated device 1-102 may be configured to reset the voltage of readout region FD of each pixel before reading out charge carriers. For example, integrated device 1-102 may be configured to reset the voltage of readout region FD, sample the voltage, transfer charge carriers into readout region FD, and sample the voltage again. In this example, the second sampled voltage may be indicative of a number of the charge carriers transferred into readout region FD when compared to the first sampled voltage. In some embodiments, integrated device 1-102 may be configured to read out charge carriers from each pixel 1-112 to the bitline sequentially, such as row by row and/or column by column. It should be appreciated that some arrays of pixels 1-112 may have multiple bitlines electrically coupled to different ones and/or groups of pixels 1-112. In some embodiments, pixels of multiple columns may be read out to respective processing circuitry at the same time. For example, a first pixel of each column (e.g., pixels (1,1) and (1,2) and so on) may be read out to the respective processing circuitry at the same time, and then a second pixel of each column (e.g., pixels (2,1) and (2,2,) and so on) may be read out to the respective processing circuitry at the same time. It should be appreciated that, in some embodiments, processing circuitry may be provided for each row of the array as an alternative or in addition to each column. In some embodiments, integrated device 1-102 may include multiple units of processing circuitry, such as each being electrically coupled to a bitline.

It should be appreciated that, in accordance with various embodiments, transfer gates described herein may include semiconductor material(s) and/or metal, and may include a gate of a field effect transistor (FET), a base of a bipolar junction transistor (BJT), and/or the like. It should also be appreciated that control signals described herein applied to the various transfer gates may vary in shape and/or voltage, such as depending on the electric potential of the semiconductor region and of the regions electrically coupled to the semiconductor region (e.g., neighboring regions).

In some embodiments, pixels described herein may include more than two charge storage regions. For example, pixel 2-112 described herein in connection with FIGS. 1-1G-1-1I includes three charge storage regions.

FIG. 1-1E is a plan view of alternative pixel 1-112', according to some embodiments. In some embodiments, pixel 1-112' may be configured in the manner described for pixel 1-112. In FIG. 1-1E, drain region D of pixel 1-112' is positioned on a same side of photodetection region PPD as charge storage regions SD0 and SD1 and readout region FD. Also shown in FIG. 1-1E, photodetection region PPD may include a mask with a triangular opening, with a base of the triangular opening on a side of photodetection region proximate charge storage regions SD0 and SD1 and drain region D, and a corresponding apex of the triangular opening on a side of photodetection region PPD opposite drain region D and charge storage regions SD0 and SD1.

In some embodiments, photodetection region PPD may be configured to induce an intrinsic electric field in a direction from photodetection region PPD toward charge storage regions SD0 and SD1 and drain region D. For example, photodetection region PPD may be formed by doping a substrate of integrated device 1-102 through the opening, resulting in a higher dopant concentration in the region of the substrate exposed through the opening than in the region covered by the mask during doping. In this example, the larger quantity of dopants (e.g., n-type dopants) at the base end of the triangular opening may cause the electric potential at the base end of photodetection region PPD proximate drain region D and charge storage region SD0 to be lower than the electric potential at the apex end of photodetection region PPD on the opposite side of photodetection region PPD. The intrinsic electric field in photodetection region PPD may be present even in the absence of an external electric field being applied to pixel 1-112. The inventors recognized that the intrinsic electric field of photodetection region PPD increases the rate of charge transfer from photodetection region PPD to drain region D and/or storage regions SD0 and SD1, increasing the efficiency with which charge carriers are drained and/or collected during operation of pixel 1-112. In the example of FIG. 1-1E, the intrinsic electric field may be directed along the dotted arrow between drain region and charge storage region SD0. For example, the intrinsic electric field may cause charge carriers to flow along the dotted arrow, and an extrinsic electric field induced by a control signal being applied to transfer gate REJ or ST0 may cause the charge carriers to flow to drain region D or charge storage region SD0, respectively.

FIG. 1-1F is a top schematic view of the pixel 1-112', according to some embodiments. As shown in FIG. 1-1F, contacts may be disposed over portions of pixel 1-112'. In some embodiments, the contacts may be configured to block incident photons from reaching portions of pixel 1-112' other than photodetection region PPD and/or from reaching photodetection regions of neighboring pixels at oblique angles of incidence. For example, the contacts may be elongated in a direction parallel to the optical axis along which photodetection PPD is configured to receive incident photons. In some embodiments, the contacts may be formed using an opaque material such as tungsten. The inventors have recognized that contacts described herein prevent many or all incident photons from reaching charge storage regions SD0 and SD1 along optical paths other than the optical axis, thereby preventing the incident photons from generating noise charge carriers in charge storage regions SD0 and SD1.

In FIG. 1-1F, a pair of contacts is disposed on opposite sides of photodetection region PPD, with a first contact of the pair disposed closer to the apex of the triangular opening of the mask and a second contact of the pair disposed closer to the base of the triangular opening of the mask. The second contact may be configured to block incident photons from reaching charge storage regions SD0 and SD1. A third contact is disposed at an end of pixel 1-112 opposite the end at which photodetection region PPD is disposed. The first and third contacts are disposed between the pixel 1-112 and respective neighboring pixels, and the second contact is positioned between photodetection region PPD and transfer gates ST0 and REJ. It should be appreciated that, in some embodiments, the pair of contacts on opposite sides of photodetection region PPD may be replaced with at least one contact wall that at least partially surrounds photodetection region PPD, such as a single cylindrical contact wall.

FIG. 1-1G is a cross-sectional view of an alternative example pixel 2-112, which may be included in integrated device 1-102, according to some embodiments. In some embodiments, pixel 2-112 may be configured in the manner described for pixel 1-112 in connection with FIGS. 1-1A-1-1F. For example, as shown in FIG. 1-1G, region FD, and drain region D, and transfer gates, each of which may be configured in the manner described for pixel 1-112. Pixel 2-112 further includes charge storage region SD2 electrically coupled between charge storage region SD1 and readout region FD. Transfer gate ST1 may electrically couple charge storage region SD0 to charge storage region SD1. For example, transfer channels may electrically couple charge storage region SD1 to charge storage region SD2 and charge storage region SD2 to readout region FD. In FIG. 1-1G, transfer gate TX0 is configured to control a transfer of charge carriers from charge storage region SD1 to charge storage region SD2, and transfer gate TX1 is configured to control a transfer of charge carriers from charge storage region SD2 to readout region FD.

FIG. 1-1H is a circuit diagram of pixel 2-112, according to some embodiments. As shown in FIG. 1-1H, the transfer channel electrically coupling charge storage region SD1 to charge storage region SD2 is a channel of a transistor having transfer gate TX0 and the transfer channel electrically coupling charge storage region SD2 to readout region FD is a channel of a transistor having transfer gate TX1. The other transistors of pixel 2-112 shown in FIG. 1-1H, such as the transistor having reset gate RST and the transistor having row select transfer gate RS may be configured in the manner described for pixel 1-112 in connection with FIGS. 1-3A and 1-3B. For example, an array of pixels 2-112 may be arranged in a configuration with processing circuitry as described herein for pixel 1-112 in connection with FIGS. 1-3B and 1-3C.

FIG. 1-1I is a top view of pixel 2-112', which may be included in integrated device 1-102, according to some embodiments. In some embodiments, pixel 2-112' may be configured in the manner described herein for pixel 1-112'. For example, photodetection region PPD of pixel 2-112' may be configured to induce an intrinsic electric field in the direction from photodetection region PPD toward charge storage region SD0 and drain region D.

### III. Integrated Device Calibration Using Photobleaching Information

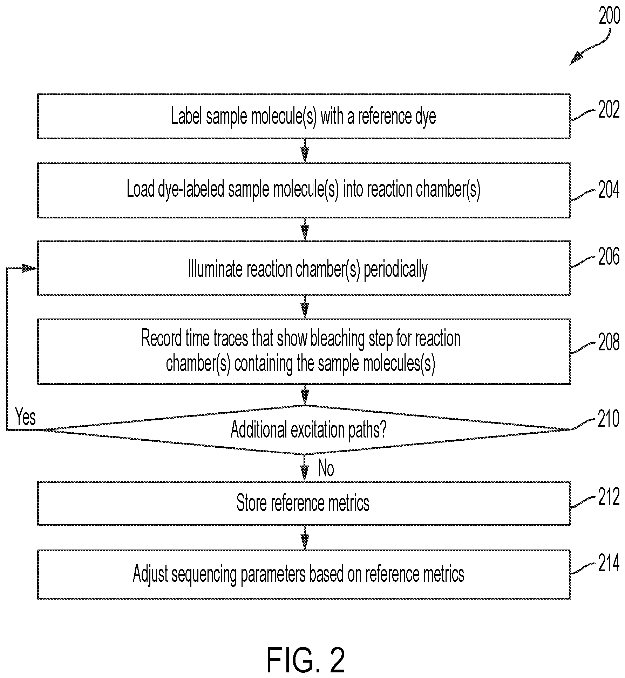

Aspects of the technology described herein relates to techniques for calibrating an integrated device and/or one or more components that interact with the integrated device using photobleaching information obtained from a reference dye. For example, the integrated device may be part of a system that further comprises at least one excitation source. The calibration techniques described herein may be used to calibrate the system comprising the integrated device. FIG. 2 illustrates an example process 200 for calibrating a system comprising an integrated device using photobleaching information, according to some embodiments.

At act 202, a sample molecule may be labeled with a reference dye. For example, the reference dye may comprise a fluorescent molecule that emits emission light in response to excitation by light from at least one light source. The reference dye molecule may be of a type that binds to a particular sample molecule such that characteristics of the emission light emitted by the reference dye molecule may be used to identify the sample molecule to which the reference dye molecule is bound.

At act 204, the dye-labeled sample is loaded into one or more reaction chambers. For example, in some embodiments, multiple reference dyes of the same type are loaded into a plurality of reaction chambers to evaluate differences in signals received from the plurality of reaction chambers. In some embodiments, the sample molecule may be labeled with the reference dye after the sample molecule is loaded into the reaction chambers, as aspects of the technology described herein are not limited in this respect.

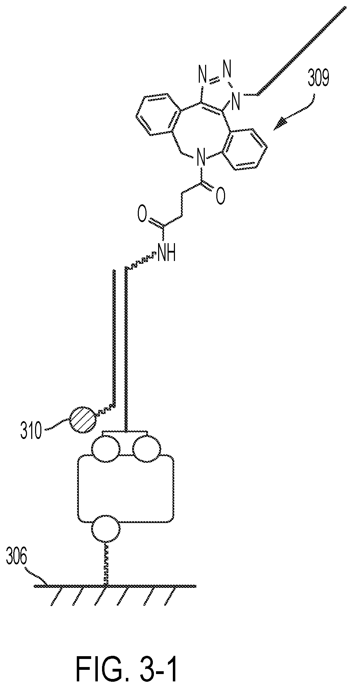

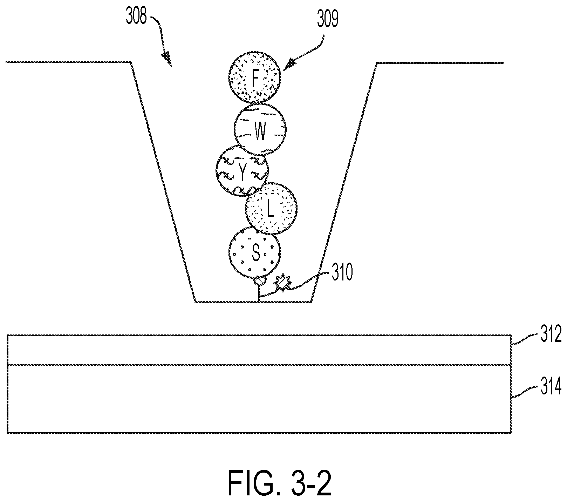

FIG. 3-1 illustrates a sample molecule 309 labeled with a reference dye 310 attached to a surface 306, according to some embodiments. FIG. 3-2 illustrates a dye-labeled sample attached in a reaction chamber, according to some embodiments. In particular, in FIG. 3-2, a sample molecule 309 comprising a chain of amino acids (phenylalanine (F), tryptophan (W), tyrosine (Y), leucine (L), and serine (S)) is loaded into the reaction chamber 308. The sample molecule 309 is labeled with a reference dye molecule 310. A CMOS chip 314 comprising photonic components 312 for collecting signals emitted by the reference dye molecule 310 may be provided, as described herein with reference to the integrated device overview.

Act 204 may be performed while the reaction chamber(s) is not being illuminated with excitation light. It should be appreciated that, in some embodiments, multiple dye-labeled sample molecules may be loaded into a plurality of reaction chambers at act 204.

In some embodiments, the sample molecule with the reference dye may be loaded into a reaction chamber alone, and a molecule to be sequenced may be loaded into the reaction chamber thereafter. In some embodiments, the molecule with the reference dye may be loaded into the reaction chamber together with the molecule to be sequenced subsequent to performing the calibration process. In some embodiments, the reference dye may be attached to the molecule to be sequenced itself, and the reference dye may be cleaved prior to performing sequencing. In some embodiments, the reference dye may be a freely diffusing dye molecule which need not be attached or otherwise immobilized in the reaction chamber. In some embodiments, the reference dye may be loaded into a reaction chamber as part of the surface chemistry preparation of the chip. Thus, aspects of the technology are not limited to the particular configuration of the reference dye in the reaction chamber. According to some embodiments, act 202 may include techniques are described in U.S. Pat. Application No. 17/082,906, filed October 28, 2020, titled "METHODS OF PREPARING SAMPLES FOR MULTIPLEX POLYPEPTIDE SEQUENCING" under Attorney Docket Number R0708.70077US01, which is incorporated by reference in its entirety.

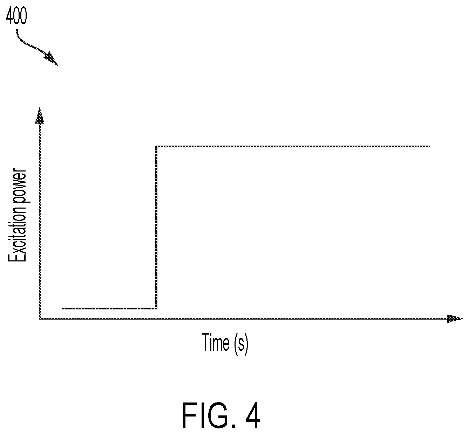

At act 206, the reaction chamber(s) is illuminated with excitation light from a light source (e.g., a laser). In particular, at act 206 the reaction chamber(s) may be illuminated with excitation light periodically (e.g., by unblocking the light source, recoupling the light source into the chip, and/or increasing the power delivered by the light source in a time that is fast relative to a characteristic bleaching time of the system). For example, FIG. 4 illustrates an example graph 400 illustrating light source excitation power over time, according to some embodiments. The excitation light may be delivered to the reaction chambers until the reference dye has been bleached in some embodiments. In some embodiments, delivery of the excitation light to the reaction chambers may be controlled to avoid bleaching the reference dye molecules. Further discussion of photobleaching reference dye molecules is provided herein.

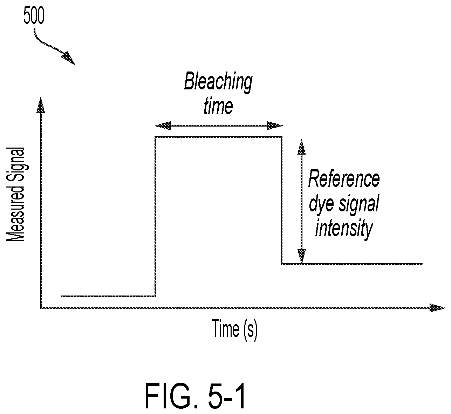

At act 208, time traces are recorded which show the bleaching step for the reaction chambers containing the sample molecule(s) illuminated at act 206. For example, FIG. 5-1 illustrates an example graph 500 illustrating measured signal from a reference dye over time, according to some embodiments. As shown in FIG. 5-1, the measured signal increases in intensity and subsequently drops in intensity after a period of time. The time period between the measured signal's rise and drop in intensity may be equated to the bleaching time of the reference dye molecule.

At act 210, it is determined whether there are additional excitation paths to excite. If, at act 210, it is determined that there are additional excitation paths to excite, the process 200 returns through the yes branch to act 206 to illuminate additional reaction chambers with the additional excitation paths. If, at act 210, it is determined that there are no additional excitation paths, the process 200 may proceed through the no branch to act 212.

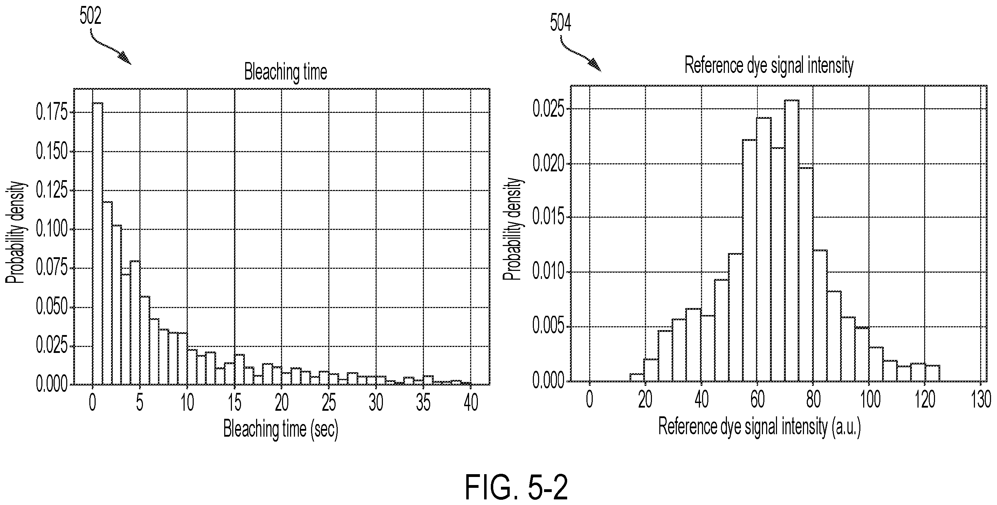

At act 212, reference metrics may be stored. For example, the reference metrics may include the time traces that show a bleaching step obtained at act 208. For example, FIG. 5-2 illustrates example histograms of measured bleaching time and dye intensity for a collection of single molecules in reaction chambers, according to some embodiments. Graph 502 illustrates a histogram of measured bleaching times for a collection of single molecule reference dyes loaded in the reaction chambers of the integrated device. As shown in FIG. 5-2, the median bleaching time for the collection of single molecule reference dyes is 5.8 seconds. Graph 504 illustrates a histogram of measured reference dye signal intensity for a collection of single molecule reference dyes loaded in the reaction chambers of the integrated device. In some embodiments, the bleaching times may be determined based at least in part on measured signal intensities. For example, the measured signal intensity may indicate a peak, and the time period between reaching the peak intensity and a drop from the peak intensity may be equated to the bleaching time.

The reference metrics may include information derived from information obtained at act 208. In some embodiments, the reference metrics may include a reference excitation intensity for molecule(s) in a reaction chamber(s) which may be determined based on the characteristic bleaching time. For example, an exponential distribution of bleaching time follows as exp(-t/τ) wherein τ is a characteristic bleaching time. For a collection of single molecules, the median bleaching time divided by ln(2) provides an estimate of the characteristic bleaching time τ. A faster characteristic bleaching time may result from a higher excitation intensity, and vice versa.

At act 214, sequencing parameters may be adjusted based on the reference metrics (e.g., characteristic bleaching time, excitation intensity). In some embodiments, the power of a laser delivering excitation light to the reaction chambers may be adjusted. For example, in some embodiments, laser power may be decreased in view of a relatively fast characteristic bleaching time. The laser power may be decreased, in some embodiments, to ensure that excitation intensity during subsequent sequencing applications does not exceed a target value. As such, the read length for sequencing applications may be improved where photodamage may be a factor in the longevity of the sequencing reaction.

In some embodiments, configuration of the integrated device may be adjusted based on the reference metrics. For example, the inventors have recognized that differences in reaction chambers may cause characteristic bleaching times of reference dye to vary between different reaction chambers. As such, the configuration of reaction chambers, photodetectors, and/or a light source delivering excitation light to the pixels may be adjusted based on the reference metrics. For example, for reaction chambers with relatively fast bleaching times, the intensity and/or duration of the excitation light delivered to the reaction chambers may be decreased.

In some embodiments, the bleaching information may be used to identify a number of reference dye molecules in a reaction chamber. For example, in embodiments where excitation light is delivered to the one or more reference dye molecules until the reference dye molecules has photobleached, a bleaching step may be observed by viewing the change in intensity of the signal collected from the reaction chamber (e.g., emitted from one or more reference dye molecules) over time. Photobleaching of one reference dye molecule may be represented by a single step in intensity (e.g., a characteristic drop in the intensity of the signal at a point in time). In some embodiments, the characteristic drop in the intensity of the signal may be substantially equal to the intensity of the signal emitted by the reference dye molecule prior to bleaching. Specifically, after bleaching, the reference dye molecule no longer emits a signal, resulting in the single step decrease in intensity substantially equal to the intensity of the signal emitted by the reference dye molecule prior to bleaching.

If a single bleaching step is observed (e.g., as represented by the change in signal intensity observed over time), it may be determined that a single reference dye molecule has bleached, and therefore that a single reference dye molecule is present in the reaction chamber from which signal is collected. If two or more bleaching steps are observed, it may be determined that multiple reference dye molecules have been loaded into a reaction chamber. The number of reference dye molecules determined to be loaded in the reaction chamber may be considered as a proxy for determining the number of biomolecules of the sample present in the reaction chamber. In some embodiments, the measure of the number of sample molecules in a reaction chamber may be used to include or exclude certain reaction chambers from subsequent analysis. Further description of using photobleaching to determine a measure of quantitative loading is provided herein.

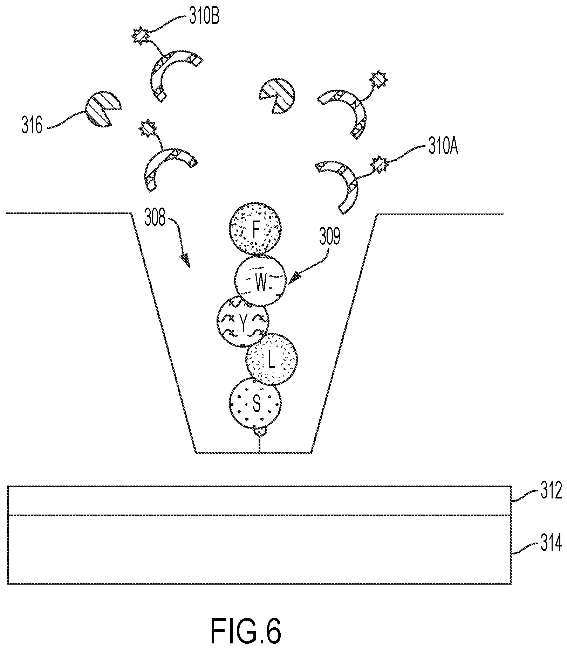

Subsequent to performing the calibration process 200, the integrated device may be used to collect and analyze signals from molecules disposed in the reaction chamber(s). For example, FIG. 6 illustrates a sequencing reaction in a reaction chamber which may be performed subsequent to the calibration process 200, according to some embodiments. The sequencing reaction shown in FIG. 6 may include attachment of reference dye molecules 310A, 310B to a sample 309 in a reaction chamber 308. As described herein, the reference dye molecules may be of a particular type, for example, binding to a particular type of sample molecule. For example, reference dye molecule 310A selectively binds only to a first type of amino acids (e.g., Leucine, denoted L in FIG. 4-1) while reference dye molecule 310B selectively binds to a second type of amino acid (e.g., Phenylalanine, Tyrosine, and Tryptophan, respectively denoted F, Y, and W in FIG. 4-1). Subsequent to sampling the sample molecule 309 (e.g., by delivering excitation light to the reaction chamber 308 and collecting signals emitted from the bound reference dye molecule 310A, 310B), cleavage of the sample molecule and/or the reference dye molecules 310A, 310B bound thereto may be performed with cleaving molecules 316. The sample molecules may be identified based on signals emitted by the reference molecules. The process of binding, receiving emission signals, and cleaving may be repeated multiple times for a single sample molecule and/or reaction chamber. In some embodiments, the calibration techniques described herein may be used to calibrate a system comprising an integrated device that is configured for use in protein and/or DNA/RNA sequencing applications.

The calibration techniques described herein may be used in combination with any suitable sampling technique to be performed subsequent to calibration. For example, in some embodiments, the calibration techniques described herein may be used in combination with techniques for sample multiplexing as described in in U.S. Pat. Application No. 63/105,185, filed October 23, 2020, titled "SYSTEMS AND METHODS FOR SAMPLE PROCESS SCALING" under Attorney Docket Number R0708.70112US00, which is incorporated by reference in its entirety.

### IV. Quantitative Loading

As described herein, some aspects relate to systems and methods for determining a measure of quantitative loading of one or more reaction chambers of the integrated device. For example, the inventors have recognized that it would be advantageous to determine in real-time (e.g., during the loading process) a measure of quantitative loading of one or more reaction chambers of the integrated device. The measure of quantitative loading may comprise an indication of how many biomolecules are present in a reaction chamber (e.g., whether the reaction chamber is empty, singly loaded, doubly loaded, or multi-loaded with more than two biomolecules). In some embodiments, the measure of quantitative loading may comprise an indication of the percentage reaction chambers being singly loaded (or any other degree loaded desired).

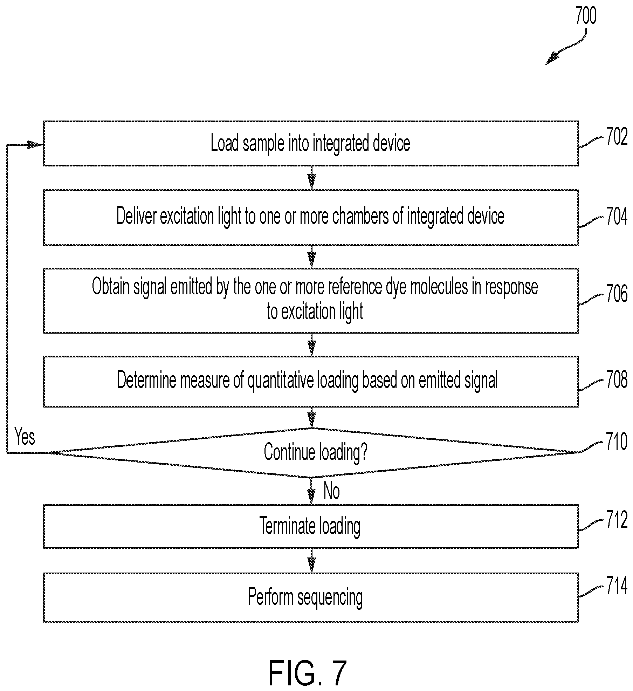

FIG. 7 illustrates an example process 700 for quantifying loading of one or more reaction chambers, according to some embodiments. The process 700 may begin at act 702 where the integrated device is loaded with a sample. The sample may comprise a plurality of biomolecules. The plurality of biomolecules may be of any suitable type. For example, the plurality of biomolecules may comprise biomolecules desired to be identified through sequencing. In some embodiments, the plurality of biomolecules comprise peptides. In some embodiments, the plurality of biomolecules comprise nucleic acids (e.g., ribonucleic acid, deoxyribonucleic acid).

The plurality of biomolecules may be labeled with fluorescent label molecules (also referred to herein as reference dye molecules). In some embodiments, the plurality of biomolecules may be labeled prior to loading the sample onto the integrated device. In some embodiments, the plurality of biomolecules may be unlabeled at the time of loading, and fluorescent label molecules previously or subsequently loaded onto the integrated device may bind to the plurality of biomolecules.

In some embodiments, a fluorescent label molecule may be attached directly to the biomolecule itself. The fluorescent label molecule may be covalently attached to the biomolecule or non-covalently attached to the biomolecule (e.g., via a linker or streptavidin). In some embodiments, the fluorescent label molecule may be loaded into the reaction chamber as part of the surface chemistry preparation of the chip. In some embodiments, the fluorescent label molecule is reversibly bound to the biomolecule, as described further herein. Thus, aspects of the technology are not limited to the particular configuration of the fluorescent label molecule in the reaction chamber. According to some embodiments, act 702 may include techniques are described in U.S. Pat. Application No. 17/082,906, filed October 28, 2020, titled "METHODS OF PREPARING SAMPLES FOR MULTIPLEX POLYPEPTIDE SEQUENCING" under Attorney Docket Number R0708.70077US01, which is incorporated by reference in its entirety.

In some embodiments, the fluorescent label molecule comprises an oligonucleotide. The oligonucleotide may be covalently attached to the biomolecule. In some embodiments, the oligonucleotide is hybridized to a complementary oligonucleotide that is covalently attached to the biomolecule.

The fluorescent label molecule may be separated from the biomolecule (e.g., via one or more spacers) by a minimum distance (e.g., more than 1 nm, more than 2 nm, more than 5 nm, 5-10 nm, more than 10 nm, 10-15 nm, more than 15 nm, 15-20 nm, more than 20 nm). Separating the fluorescent label molecule from the biomolecule by a minimum distance may prevent damage occurring to the biomolecule during the quantitative loading determination (e.g., during photobleaching).

At act 704, excitation light may be delivered to one or more reaction chambers of the integrated device. The excitation light may be generated via at least one excitation source (e.g., a laser), as described herein. The excitation light may be delivered to all of the reaction chambers of the integrated device or a portion thereof.

The excitation light causes any fluorescent label molecules bound to biomolecules in the reaction chambers to excite and emit excitation light. At act 706, a signal emitted by the one or more reference dye molecules from the respective reaction chambers in response to the excitation light may be obtained.

For example, the emission light may be collected by one or more photodetection regions of the integrated device. In some embodiments, the integrated device may include one photodetection region for each reaction chamber. In some embodiments, multiple photodetection regions may be provided for a single reaction chamber. In some embodiments, multiple reaction chambers may correspond to a single photodetection region.

At act 708, the measure of quantitative loading may be determined based on the emitted signal obtained at act 706. As described herein, the measure of quantitative loading may comprise an indication of how many biomolecules are present in a reaction chamber (e.g., whether the reaction chamber is empty, singly loaded, doubly loaded, or multi-loaded with more than two biomolecules). In some embodiments, the measure of quantitative loading may comprise an indication of the percentage reaction chambers being singly loaded (or any other degree loaded desired).

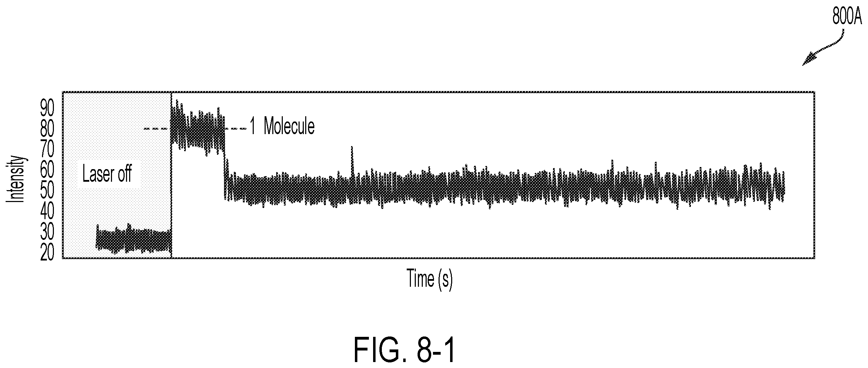

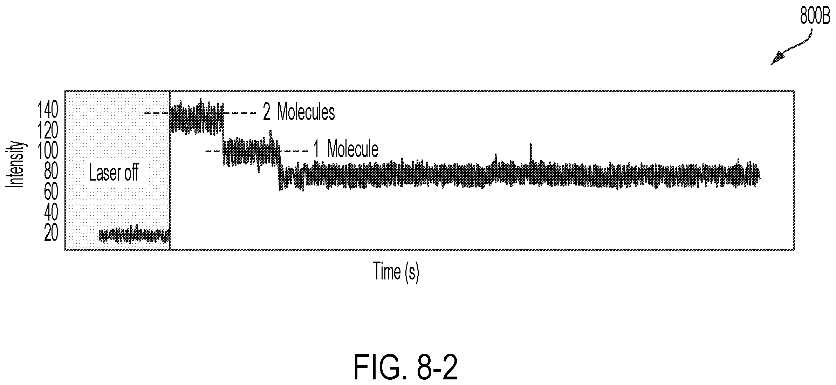

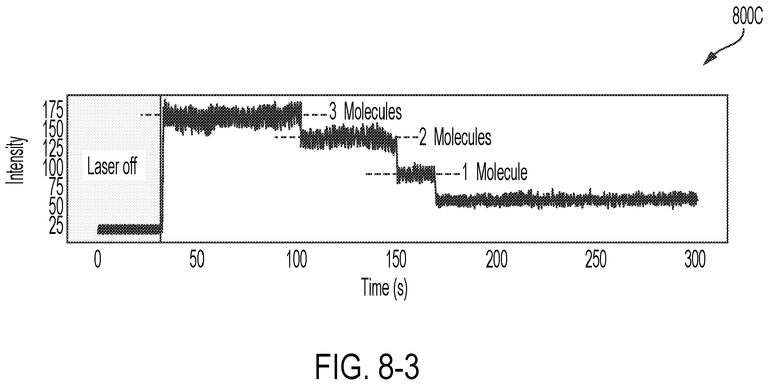

In some embodiments, delivering the excitation light to the one or more reaction chambers of the integrated device comprises photobleaching the one or more fluorescent label molecules, and the measure of quantitative loading is determined based on a number of photobleaching steps represented in the emitted signal (e.g., a number of photobleaching steps that may be observed in the emitted signal). For example, the reaction chamber(s) may be illuminated with excitation light as a step function (e.g., by unblocking the light source, recoupling the light source into the chip, and/or increasing the power delivered by the light source in a time that is fast relative to a characteristic bleaching time of the system). The excitation light may be delivered to the reaction chambers until the reference dye has been bleached. FIGS. 8-1, 8-2, and 8-3 illustrates example traces obtained from chambers having fluorescent label molecules being photobleached at act 704. In other embodiments, signals emitted by the one or more reference dyes in the reaction chambers may be obtained without performing photobleaching.

FIG. 8-1 illustrates an example trace 800A from a chip loading process that represents a single loaded well, according to some embodiments. As shown in FIG. 8-1, signal intensity over a period of time is plotted. When excitation light is first delivered to the reaction chamber, the intensity of a signal received from the reaction chamber may rise to a peak. Subsequent to delivering excitation light to the reaction chambers (e.g., by turning a light source, such as a laser, on), a single step in intensity (e.g., a characteristic drop in the intensity of the signal at a point in time) can be seen in FIG. 8-1. In some embodiments, as depicted in FIG. 8-1, the characteristic drop in the intensity of the signal may be substantially equal to the intensity of the signal emitted by the reference dye molecule prior to bleaching. After bleaching, the reference dye molecule no longer emits a signal, resulting in the single step decrease in intensity shown in FIG. 5-1. Accordingly, the signal shown in FIG. 8-1 indicates the presence of a single fluorescent label in the reaction chamber and therefore a single biomolecule.

FIG. 8-2 illustrates an example trace 800B from a chip loading process that represents a doubly loaded well, according to some embodiments. As shown in FIG. 8-2, the signal comprises two steps in signal intensity indicating the presence of two fluorescent label molecules and therefore two biomolecules in the reaction chamber.

FIG. 8-3 illustrates an example trace 800C from a chip loading process that represents a multi-loaded well, according to some embodiments. As shown in FIG. 8-3, the signal comprises three steps in signal intensity (three characteristic drops in intensity of the received signal) indicating the presence of three fluorescent label molecules and therefore three biomolecules in the reaction chamber.

In some instances, no photobleaching steps may be observed. Accordingly, it may be determined, based on the lack of photobleaching observed, that no biomolecules are present in the reaction chamber.

In some embodiments, a signal may be obtained from the reaction chamber without photobleaching fluorescent label molecules present in the reaction chamber. In such embodiments, the number of fluorescent label molecules, and therefore the number of biomolecules, present in the reaction chamber may be determined by a relative intensity of the signal obtained from the reaction chamber.

In some embodiments, the fluorescent label molecule may reversibly bind to the biomolecule or a secondary biomolecule attached there to. For example, the secondary biomolecule may comprise an N-terminal amino acid recognizer (e.g., a ClpS, UBR box, etc.). In some embodiments, the secondary biomolecule is an oligonucleotide. In some embodiments, the oligonucleotide is capable of reversible hybridization to the biomolecule or an oligonucleotide attached thereto.

The binding on and off of the fluorescent label molecule to the biomolecule may be expressed by the on and off pulsing of the fluorescent label molecule. In particular, when the fluorescent label molecule is bound to the biomolecule and excited with excitation light, the fluorescent label molecule emits emission light that may be collected by one or more photodetection regions. When not bound, the fluorescent label molecule may not emit emission light. The fluorescent label molecule may follow a periodic binding pattern to the biomolecule. As such, the fluorescent label molecule generates a periodic pulsing pattern that can be detected by collecting the light emitted from the fluorescent label molecule when bound to a biomolecule.

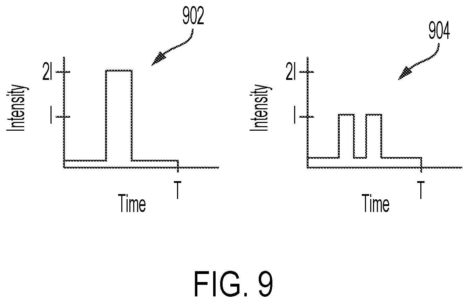

FIG. 9 illustrates a periodic pulsing pattern of fluorescent molecules reversibly bound to a biomolecule, according to some embodiments. Plots 902 and 904 illustrate examples of pulses detected by one or more photodetection regions during a single pulsing period T.

Plots 902 and 904 illustrate examples of a doubly loaded well having two biomolecules and respective fluorescent labels reversibly bound thereto. Therefore two pulses are detected during the pulsing period T. In the illustrated embodiment of plot 902, the two fluorescent label molecules bind to the respective biomolecules at substantially the same time during period T. Therefore, a single composite pulse comprised of respective pulses from each of the two fluorescent label molecules is detected in the period T, having intensity 2I. In the illustrated embodiment of plot 904, the two fluorescent label molecules bind to the respective biomolecules at discrete times during period T. Accordingly, two pulses having intensity I are detected in the period T.

Although the illustrated embodiments give examples where a reaction chamber is doubly loaded with two biomolecules, the techniques described herein may likewise be used to detect the presence of a single biomolecule, more than two biomolecules, or the lack of any biomolecules in a reaction chamber by determining the intensity of pulses emitted from the reaction chamber during a pulsing period T. The pulsing period and intensity of the pulses may be determined based on a characteristic pulse width and intensity of the fluorescent label molecule being reversibly bound to the biomolecules.

FIG. 9 illustrates how the timing and intensity of pulses can be detected and used to determine a number of fluorescent label molecules in a reaction chamber being bound to a biomolecule during a respective pulsing period T, and therefore, a number of biomolecules currently present in a reaction chamber. Accordingly, act 708 of process 700 may be performed using reversible binding molecules as described herein.

Turning back to process 700, the process 700 may proceed, after determining the measure of quantitative loading based on the emitted signal, to act 710. At act 710 it is determined whether to continue loading. The determination to continue or terminate loading may be determined based on the measure of quantitative loading determined at act 710.

For example, in some embodiments, operation of the integrated device may be optimized based on the measure of quantitative loading. In some embodiments, the measure of quantitative loading may be used to adjust how loading of the sample onto the integrated device is performed. In some embodiments, the measure of quantitative loading may be used to determine whether an optimal number of reaction chambers have been loaded with a desired number of biomolecules. In some embodiments, the desired number of biomolecules is one, such that the measure of quantitative loading is used to determine whether an optimal number (e.g., a maximum) of reaction chambers are singly loaded.

If the optimal number of loaded reaction chambers has not been reached, as indicated by the measure of quantitative loading, additional loading may be performed. For example, the process 700 may loop back to act 702 where additional sample is loaded onto the integrated device.

In some embodiments, the measure of quantitative loading may be used to adjust an amount of additional sample that is loaded onto the integrated device at subsequent loading steps. For example, the sample may comprise a particular concentration of biomolecules. The measure of quantitative loading may be used to determine whether to increase or decrease the concentration of biomolecules. For example, if it is determined that the optimal number of singly loaded reaction chambers has not been met, additional loading may be performed with an additional sample having a higher concentration of biomolecules than the initial sample.

In some embodiments, the measure of quantitative loading may be used to adjust how signals from the reaction chambers are processed for subsequent analysis. For example, when a reaction chamber is determined to be empty, doubly loaded, or multi-loaded, it may be determined to disregard signals from these reaction chambers either by not performing sequencing on these reaction chambers or not processing signals received from these reaction chambers.

In some embodiments, the measure of quantitative loading may be used to inform future operation of the integrated device. For example, the measure of quantitative loading may be used to adjust how loading of a sample onto the integrated device is performed in the future (e.g., what concentration of biomolecules is present in the sample, how long to perform loading, at what rate to perform loading, etc.).

When it is determined not to continue loading at act 710, the process 700 may proceed to act 712 where loading is terminated. For example, in some embodiments, termination may be performed by removing, washing, and/or replacing of the solution containing non-immobilized (e.g., unbound) peptides. In some embodiments, termination may be performed autonomously.

In some embodiments, terminating loading may include removing the fluorescent label molecule from the reaction chamber. In some embodiments, the fluorescent label molecule may be removed by a chemical cleavage process. In some embodiments, the fluorescent label molecule may be removed by an enzymatic cleavage process

As described herein, the process 700 may be performed continuously. In some embodiments, the process 700 may be performed autonomously, for example, using software. This may allow for continuous monitoring of the number of labeled biomolecules in each reaction chamber. A software analysis process may monitor the loading in real time. For example, the software analysis may detect signal pulses/photobleaching steps, as described herein.

In some embodiments, one or more different types of fluorescent label molecules are provided to the biomolecules. For example, a first type of fluorescent label molecule and a second type of fluorescent label molecule may be provided for binding to the biomolecules.

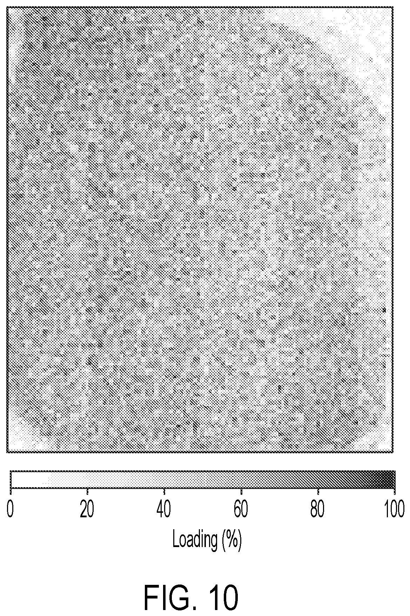

In some embodiments, may generate a "heatmap" of reaction chambers to visually illustrate a percentage of the integrated device (e.g., a percentage of reaction chambers) loaded with one or more biomolecules. FIG. 10 illustrates an example heatmap of reaction chambers illustrating percent loading, according to some embodiments.

In some embodiments, the measure of quantitative loading may only be obtained for a portion of the integrated device (e.g., a portion of all reaction chambers of the integrated device). The measure of quantitative loading may be extrapolated to the remaining reaction chambers of the integrated device to obtain an extrapolated measure of quantitative loading for the entire integrated device (or other portions therefore).

In some embodiments, sequencing of the sample may be performed subsequent to performing the calibration and quantitative loading techniques described herein (e.g., at act 714 of process 700). For example, the calibration and quantitative loading techniques may be used in combination with techniques for sample identification using machine learning, for example, as described in in U.S. Pat. Application No. 16/900,582, filed June 12, 2020, titled "TECHNIQUES FOR PROTEIN IDENTIFICATION USING MACHINE LEARNING AND RELATED SYSTEMS AND METHODS" under Attorney Docket Number R0708.70063US01, which is incorporated by reference in its entirety.

### V. Equivalents and Scope

Having thus described several aspects and embodiments of the technology of the present disclosure, it is to be appreciated that various alterations, modifications, and improvements will readily occur to those of ordinary skill in the art. Such alterations, modifications, and improvements are intended to be within the spirit and scope of the technology described herein. It is, therefore, to be understood that the foregoing embodiments are presented by way of example only and that, within the scope of the appended claims and equivalents thereto, inventive embodiments may be practiced otherwise than as specifically described. In addition, any combination of two or more features, systems, articles, materials, kits, and/or methods described herein, if such features, systems, articles, materials, kits, and/or methods are not mutually inconsistent, is included within the scope of the present disclosure.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. The transitional phrases "consisting of" and "consisting essentially of' shall be closed or semi-closed transitional phrases, respectively.

### EMBODIMENTS OF THE INVENTION

1. A method for calibrating a system comprising an integrated device, the method comprising:
   exciting, with light from at least one excitation source, a reference dye molecule disposed in a chamber of the integrated device;
   obtaining a signal emitted by the reference dye molecule, the signal containing information representative of a bleaching time of the reference dye molecule; and
   adjusting one or more characteristics of the system based on the bleaching time of the reference dye molecule.
2. The method of embodiment 1, wherein the one or more characteristics comprise a power of the at least one excitation source.
3. The method of embodiment 2, wherein the adjusting comprises decreasing the power of the at least one excitation source when the bleaching time of the reference dye molecule is less than a threshold time.
4. The method of any one of embodiments 1-3, wherein exciting the reference dye molecule comprises delivering light to the chamber of the integrated device at least until the reference dye molecule undergoes photobleaching.
5. The method of any one of embodiments 1-4, further comprising determining the bleaching time of the reference dye molecule based on the signal emitted by the reference dye molecule.
6. The method of embodiment 5, wherein determining the bleaching time of the reference dye molecule comprises determining a duration in which an intensity of the signal emitted by the reference dye molecule exceeds a threshold intensity.
7. The method of any one of embodiments 1-6, further comprising:
   subsequent to adjusting the one or more characteristics of the system, operating the integrated device to obtain a signal emitted by a sample disposed in the chamber of the integrated device, wherein the signal emitted by the sample comprises information representative of at least one characteristic of the sample.
8. The method of embodiment 7, further comprising identifying the sample based on the at least one characteristic of the sample.
9. The method of embodiment 8, wherein the sample comprises a polypeptide.
10. The method of embodiment 9, wherein identifying the sample comprises identifying one or more amino acids of the sample based at least in part on the information representative of the at least one characteristic of the sample, and identifying the polypeptide based at least in part on the one or more amino acids.
11. The method of embodiment 8, wherein the sample comprises a nucleic acid strand.
12. The method of embodiment 8, wherein identifying the sample comprises identifying one or more nucleotides of the nucleic acid strand based at least in part on the information representative of the at least one characteristic of the sample.
13. The method of any one of embodiments 1-12, wherein:
   the chamber comprises a plurality of chambers;
   the reference dye molecule comprises a plurality of reference dye molecules disposed in respective ones of the plurality of chambers; and
   obtaining a signal emitted by the reference dye molecule comprises obtaining a plurality of signals emitted by the plurality of reference dye molecules; and
   adjusting the one or more characteristics of the system is based on information representative of bleaching times of the plurality of reference dye molecules.
14. The method of embodiment 13, wherein the plurality of reference dye molecules comprises a same molecule.
15. An integrated device comprising:
   at least one chamber for receiving a reference dye molecule;
   at least one photodetection region for receiving a signal emitted by the reference dye molecule when excited by light from at least one excitation source, the signal containing information representative of a bleaching time of the reference dye molecule; and
   at least one controller configured to control adjusting of one or more characteristics of a system comprising the integrated device based on the bleaching time of the reference dye molecule.
16. The integrated device of embodiment 15, wherein the one or more characteristics comprise a power of the at least one excitation source.
17. The integrated device of embodiment 16, wherein the at least one controller is configured to control the adjusting of the one or more characteristics of the system at least in part by decreasing the power of the at least one excitation source when the bleaching time of the reference dye molecule is less than a threshold time.
18. The integrated device of any one of embodiments 15-17, wherein the at least one controller is further configured to determine the bleaching time of the reference dye molecule.
19. The integrated device of embodiment 18, wherein the at least one controller is configured to determine the bleaching time of the reference dye molecule at least in part by determining at duration in which an intensity of the signal emitted by the reference dye molecule exceeds a threshold intensity.
20. A method of manufacturing an integrated device, comprising:
   providing a chamber on a substrate of the integrated device, the chamber being configured for receiving a reference dye molecule and positioned on the substrate such that the reference dye molecule receives light from at least one light source;
   providing a photodetection region positioned adjacent to the chamber such that the photodetection region receives a signal emitted by the reference dye molecule when the light from the at least one light source is delivered to the reference dye molecule; and
   coupling at least one controller to the photodetection region so that the at least one controller receives information contained by the signal emitted by the reference dye molecule, the information being representative of a bleaching time of the reference dye molecule.
21. A method for determining a measure of quantitative loading of a sample in an integrated device, the method comprising:
   exciting, with excitation light from at least one excitation source, one or more reference dye molecules that, during the exciting with the excitation light, are attached to respective biomolecules of the sample bound to a surface of a chamber of one or more chambers of the integrated device;
   obtaining a signal emitted by the one or more reference dye molecules in response to the excitation light; and
   determining, based on the signal emitted by the one or more reference dye molecules, the measure of quantitative loading of the sample.
22. The method of embodiment 21, wherein the signal expresses an intensity of light emitted by the one or more reference dye molecules over a period of time.
23. The method of any one of embodiments 21-22, wherein the exciting the one or more reference dye molecules comprises photobleaching the one or more reference dye molecules.
24. The method of embodiment 23, further comprising determining a number of photobleaching steps in the signal emitted by the one or more reference dye molecules and determining a number of respective biomolecules that are bound to the surface of the chamber based on the number of photobleaching steps.
25. The method of any one of embodiments 21-24, wherein the measure of quantitative loading of the sample comprises a number of the respective biomolecules bound to a surface of a single chamber of the one or more chambers.
26. The method of any one of embodiments 21-25, wherein the measure of quantitative loading of the sample comprises a percentage of the one or more chambers containing a single biomolecule of the sample bound to a surface of a respective one of the one or more chambers.
27. The method of any one of embodiments 21-26, further comprising optimizing operation of the integrated device based on the measure of quantitative loading.
28. The method of embodiment 27, wherein optimizing operation of the integrated device based on the measure of quantitative loading comprises adjusting how loading of the sample onto the integrated device is performed.
29. The method of embodiment 28, wherein adjusting how loading of the sample onto the integrated device is performed comprises adjusting a concentration of biomolecules in the sample.
30. The method of embodiment 28, wherein adjusting how loading of the sample is performed comprises adjusting how loading of the sample is performed to maximize a number of the one or more chambers that contain a single biomolecule bound to a surface thereof.
31. The method of embodiment 27, wherein optimizing operation of the integrated device based on the measure of quantitative loading comprises excluding signals from at least some of the one or more chambers from subsequent analysis.
32. The method of any one of embodiments 21-31, further comprising reloading the one or more chambers with additional sample until an optimal number of the one or more chambers each contain at least one and no more than one biomolecule bound to a surface of a respective one of the one or more chambers.
33. The method of embodiment 32, further comprising using the measure of quantitative loading to determine when the optimal number of the one or more chambers each contain at least one and no more than one biomolecule bound to a surface of a respective one of the one or more chambers.
34. The method of embodiment 33, further comprising terminating loading when the optimal number of the one or more chambers each contain at least one and no more than one biomolecule of the sample, wherein terminating loading comprises removing unbound biomolecules from the integrated device.
35. The method of embodiment 34, further comprising subsequent to terminating loading, sequencing the sample at least in part by:
   delivering excitation light to the one or more chambers;
   obtaining signals emitted from the one or more chambers in response to the excitation light; and
   identifying one or more biomolecules of the sample based on the signals.
36. The method of any one of embodiments 21-35, wherein each of the one or more reference dye molecules, when attached to the respective biomolecule, is separated from the respective biomolecule by at least 1 nm.
37. The method of any one of embodiments 21-36, wherein the respective biomolecule is one of a peptide or a nucleic acid.
38. The method of any one of embodiments 21-37, wherein the one or more reference dye molecules are covalently linked to the respective biomolecule.
39. The method of any one of embodiments 21-38, wherein the one or more reference dye molecules are non-covalently linked to the respective biomolecule.
40. The method of any one of embodiments 21-39, wherein the one or more reference dye molecules are bound to a respective secondary biomolecule, and the respective secondary biomolecule is attached to the respective biomolecules of the sample.
41. The method of embodiment 40, wherein the respective secondary biomolecule comprises an oligonucleotide.
42. The method of embodiment 40, wherein the respective secondary biomolecule comprises an N-terminal amino acid recognizer.
43. The method of embodiment 40, wherein the respective secondary biomolecule reversibly binds to the respective biomolecule.
44. The method of embodiment 43, wherein the one or more reference dye molecules exhibit a pulsing pattern, such that, a respective reference dye molecule of the one or more reference dye molecules emits emission light only when bound to the respective biomolecule.
45. The method of embodiment 44, wherein the measure of quantitative loading of the sample comprises a number of biomolecules bound to the surface of the chamber and the method further comprises determining the number of biomolecules in the chamber based on the pulsing pattern of the one or more reference dye molecules.
46. The method of any one of embodiments 21-45, wherein at least some of the one or more reference dye molecules are attached to a respective linker to which the respective biomolecule is bound, wherein the respective linker is bound to the surface of the chamber.
47. An integrated device configured to determine a measure of quantitative loading of a sample, the integrated device comprising:
   at least one chamber for receiving one or more reference dye molecules that, during excitation of the one or more reference dye molecules with excitation light delivered from at least one excitation source, are attached to respective biomolecules of the sample, the respective biomolecules being bound to a surface of the at least one chamber;
   at least one photodetection region for receiving a signal emitted by the one or more reference dye molecules in response to the excitation light from the at least one excitation source; and
   at least one controller configured to determine, based on the signal emitted by the one or more reference dye molecules, the measure of quantitative loading of the sample.
48. The integrated device of embodiment 47, wherein the signal expresses an intensity of light emitted by the one or more reference dye molecules over a period of time.
49. The integrated device of any one of embodiments 47-48, wherein the at least one controller is configured to control the at least one excitation source to deliver the excitation light to the at least one chamber at least until the one or more reference dye molecules photobleach.
50. The integrated device of embodiment 49, wherein the at least one controller is configured to determine a number of photobleaching steps in the signal emitted by the one or more reference dye molecules and to determine a number of respective biomolecules bound to the surface of the chamber based on the number of photobleaching steps.
51. The integrated device of any one of embodiments 47-50, wherein the measure of quantitative loading of the sample comprises a number of the respective biomolecules bound to a surface of a single chamber of the at least one chamber.
52. The integrated device of any one of embodiments 47-51, wherein the measure of quantitative loading of the sample comprises a percentage of chambers of the at least one chamber containing a single biomolecule of the sample bound to a surface of a respective chamber of the at least one chamber.
53. The integrated device of any one of embodiments 47-52, wherein the at least one controller is further configured to determine, using the measure of quantitative loading, when an optimal number of chambers of the at least one chamber each contain at least one and no more than one biomolecule bound to a surface of a respective chamber of the at least one chamber.
54. The integrated device of any one of embodiments 47-53, wherein the at least one controller is further configured to identify the respective biomolecule based on the signal.
55. The integrated device of any one of embodiments 47-54, wherein the respective biomolecule is one of a peptide or a nucleic acid.
56. The integrated device of any one of embodiments 47-55, wherein the one or more reference dye molecules are covalently linked to the respective biomolecules.
57. The integrated device of any one of embodiments 47-55, wherein the one or more reference dye molecules are non-covalently linked to the respective biomolecule.
58. The integrated device of any one of embodiments 47-56, wherein the one or more reference dye molecules are bound to a respective secondary biomolecule, and the respective secondary biomolecule is attached to the respective biomolecules of the sample.
59. The integrated device of embodiment 58, wherein the respective secondary biomolecule comprises an oligonucleotide.
60. The integrated device of embodiment 58, wherein the respective secondary biomolecule comprises an N-terminal amino acid recognizer.
61. The integrated device of embodiment 58, wherein the respective secondary biomolecule reversibly binds to the respective biomolecule.
62. A method for determining a measure of quantitative loading of a sample in an integrated device, the method comprising:
   exciting, with light from at least one excitation source, one or more reference dye molecules, the one or more reference dye molecules being attached to respective secondary biomolecules which reversibly binds to respective biomolecules of the sample, the respective biomolecules being bound to a surface of a chamber of a plurality of chambers of the integrated device;
   obtaining a signal emitted by the one or more reference dye molecules in response to the excitation light;
   determining a pulsing pattern of the one or more reference dye molecules; and
   determining the measure of quantitative loading of the sample based on the pulsing pattern of the one or more reference dye molecules.
63. The method of embodiment 62, wherein:
   the respective secondary biomolecules binds to the respective biomolecules of the sample once per pulsing period;
   the pulsing pattern comprises one or more pulses or no pulses; and
   the measure of quantitative loading is determined based on a number of the one or more pulses received during a pulsing period.
64. The method of any one of embodiments 62-63, wherein the respective secondary biomolecules comprise oligonucleotides.
65. The method of any one of embodiments 62-64, wherein the respective secondary biomolecules comprise an N-terminal amino acid recognizers.
66. The method of embodiment 63, wherein the measure of quantitative loading of the sample comprises a number of the respective biomolecules of the sample bound to the surface of the chamber.
67. The method of embodiment 66, wherein determining the measure of quantitative loading of the sample comprises, when the no pulses are present in the pulsing pattern, determining that the chamber contains no biomolecules.
68. The method of any one of embodiments 62-67, wherein the signal expresses an intensity of light emitted by the one or more reference dye molecules over a period of time.
69. The method of any one of embodiments 62-68, wherein the exciting the one or more reference dye molecules comprises photobleaching the one or more reference dye molecules.
70. The method of embodiment 69, further comprising determining a number of photobleaching steps in the signal emitted by the one or more reference dye molecules and determining a number of respective biomolecules that are bound to the surface of the chamber based on the number of photobleaching steps.
71. The method of any one of embodiments 62-70, wherein the measure of quantitative loading of the sample comprises a number of the respective biomolecules bound to a surface of a single chamber of the one or more chambers.
72. The method of any one of embodiments 62-71, wherein the measure of quantitative loading of the sample comprises a percentage of the one or more chambers containing a single biomolecule of the sample bound to a surface of a respective one of the one or more chambers.
73. The method of any one of embodiments 62-72, further comprising optimizing operation of the integrated device based on the measure of quantitative loading.
74. The method of embodiment 73, wherein optimizing operation of the integrated device based on the measure of quantitative loading comprises adjusting how loading of the sample onto the integrated device is performed.
75. The method of embodiment 74, wherein adjusting how loading of the sample onto the integrated device is performed comprises adjusting a concentration of biomolecules in the sample.
76. The method of embodiment 74, wherein adjusting how loading of the sample is performed comprises adjusting how loading of the sample is performed to maximize a number of the one or more chambers that contain a single biomolecule bound to a surface thereof.
77. The method of embodiment 74, wherein optimizing operation of the integrated device based on the measure of quantitative loading comprises excluding signals from at least some of the one or more chambers from subsequent analysis.
78. The method of any one of embodiments 62-77, further comprising reloading the one or more chambers with additional sample until an optimal number of the one or more chambers each contain at least one and no more than one biomolecule bound to a surface of a respective one of the one or more chambers.
79. The method of embodiment 78, further comprising using the measure of quantitative loading to determine when the optimal number of the one or more chambers each contain at least one and no more than one biomolecule bound to a surface of a respective one of the one or more chambers.
80. The method of embodiment 78, further comprising terminating loading when the optimal number of the one or more chambers each contain at least one and no more than one biomolecule of the sample, wherein terminating loading comprises removing unbound biomolecules from the integrated device.
81. The method of embodiment 80, further comprising subsequent to terminating loading, sequencing the sample at least in part by:
   delivering excitation light to the one or more chambers;
   obtaining signals emitted from the one or more chambers in response to the excitation light; and
   identifying one or more biomolecules of the sample based on the signals.

## Claims

1. A method for determining a measure of quantitative loading of a sample in an
integrated device, the method comprising:
exciting, with excitation light from at least one excitation source, one or more reference dye molecules that, during the exciting with the excitation light, are attached to respective biomolecules of the sample bound to a surface of a chamber of one or more chambers of the integrated device;
obtaining a signal emitted by the one or more reference dye molecules in response to the excitation light; and
determining, based on the signal emitted by the one or more reference dye molecules, the measure of quantitative loading of the sample.

2. A method for determining a measure of quantitative loading of a sample in an integrated device, the method comprising:
exciting, with light from at least one excitation source, one or more reference dye molecules, the one or more reference dye molecules being attached to respective secondary biomolecules which reversibly binds to respective biomolecules of the sample, the respective biomolecules being bound to a surface of a chamber of a plurality of chambers of the integrated device;
obtaining a signal emitted by the one or more reference dye molecules in response to the excitation light;
determining a pulsing pattern of the one or more reference dye molecules; and
determining the measure of quantitative loading of the sample based on the pulsing pattern of the one or more reference dye molecules.

3. An integrated device configured to determine a measure of quantitative loading of a sample, the integrated device comprising:
at least one chamber for receiving one or more reference dye molecules that, during excitation of the one or more reference dye molecules with excitation light delivered from at least one excitation source, are attached to respective biomolecules of the sample, the respective biomolecules being bound to a surface of the at least one chamber;
at least one photodetection region for receiving a signal emitted by the one or more reference dye molecules in response to the excitation light from the at least one excitation source; and
at least one controller configured to determine, based on the signal emitted by the one or more reference dye molecules, the measure of quantitative loading of the sample.

4. The method of claims 1 or 2, or the integrated device of claim 3, wherein the signal expresses an intensity of light emitted by the one or more reference dye molecules over a period of time.

5. The method of claims 1, 2 or 4, or the integrated device of claims 3-4, wherein the exciting the one or more reference dye molecules comprises photobleaching the one or more reference dye molecules, optionally further comprising determining a number of photobleaching steps in the signal emitted by the one or more reference dye molecules and determining a number of respective biomolecules that are bound to the surface of the chamber based on the number of photobleaching steps.

6. The method of claims 1, 2, 4 or 5, or the integrated device of any one of claims 3-5, wherein the measure of quantitative loading of the sample comprises a number of the respective biomolecules bound to a surface of a single chamber of the one or more chambers, and/or wherein the measure of quantitative loading of the sample comprises a percentage of the one or more chambers containing a single biomolecule of the sample bound to a surface of a respective one of the one or more chambers.

7. The method of any one of claims 1, 2, 4-6, further comprising optimizing operation of the integrated device based on the measure of quantitative loading, optionally wherein optimizing operation of the integrated device based on the measure of quantitative loading comprises either: excluding signals from at least some of the one or more chambers from subsequent analysis, or adjusting how loading of the sample onto the integrated device is performed, optionally wherein adjusting how loading of the sample onto the integrated device is either: performed to maximize a number of the one or more chambers that contain a single biomolecule bound to a surface thereof, or comprises adjusting a concentration of biomolecules in the sample.

8. The method of any one of claims 1, 2, 4-7, further comprising reloading the one or more chambers with additional sample until an optimal number of the one or more chambers each contain at least one and no more than one biomolecule bound to a surface of a respective one of the one or more chambers, optionally further comprising using the measure of quantitative loading to determine when the optimal number of the one or more chambers each contain at least one and no more than one biomolecule bound to a surface of a respective one of the one or more chambers.

9. The method of claim 8, further comprising terminating loading when the optimal number of the one or more chambers each contain at least one and no more than one biomolecule of the sample, wherein terminating loading comprises removing unbound biomolecules from the integrated device, optionally further comprising subsequent to terminating loading, sequencing the sample at least in part by: delivering excitation light to the one or more chambers; obtaining signals emitted from the one or more chambers in response to the excitation light; and identifying one or more biomolecules of the sample based on the signals.

10. The method of any one of claims 1, 2, 4-9, or the integrated device of any one of claims 3-6, wherein each of the one or more reference dye molecules, when attached to the respective biomolecule, is separated from the respective biomolecule by at least 1 nm, and/or wherein the one or more reference dye molecules are covalently linked to the respective biomolecule, or wherein the one or more reference dye molecules are non-covalently linked to the respective biomolecule.

11. The method of any one of claims 1, 2, 4-10, or the integrated device of any one of claims 3-6, 9 or 10, wherein the one or more reference dye molecules are bound to a respective secondary biomolecule, and the respective secondary biomolecule is attached to the respective biomolecules of the sample, optionally wherein the respective secondary biomolecule comprises an oligonucleotide or an N-terminal amino acid recognizer, and/or wherein the respective secondary biomolecule reversibly binds to the respective biomolecule.

12. The method or the integrated device of claim 11, wherein the one or more reference dye molecules exhibit a pulsing pattern, such that, a respective reference dye molecule of the one or more reference dye molecules emits emission light only when bound to the respective biomolecule, optionally wherein the measure of quantitative loading of the sample comprises a number of biomolecules bound to the surface of the chamber and the method further comprises determining the number of biomolecules in the chamber based on the pulsing pattern of the one or more reference dye molecules.

13. The method of any one of claims 1, 2, 4-12 or the integrated device of any one of claims 3-6, or 10-12, wherein at least some of the one or more reference dye molecules are attached to a respective linker to which the respective biomolecule is bound, wherein the respective linker is bound to the surface of the chamber.

14. The integrated device of any one of claims 3-6 or 10-13, wherein the at least one controller is further configured to identify the respective biomolecule based on the signal.

15. The method of claim 2, wherein:
the respective secondary biomolecules binds to the respective biomolecules of the sample once per pulsing period;
the pulsing pattern comprises one or more pulses or no pulses; and
the measure of quantitative loading is determined based on a number of the one or more pulses received during a pulsing period, optionally wherein the measure of quantitative loading of the sample comprises a number of the respective biomolecules of the sample bound to the surface of the chamber, further optionally wherein determining the measure of quantitative loading of the sample comprises, when the no pulses are present in the pulsing pattern, determining that the chamber contains no biomolecules.
